# EUROPEAN PATENT SPECIFICATION

(11) **EP 3 034 581 B1**
(45) Date of publication and mention of the grant of the patent: **04.04.2018**
(21) Application number: 14836457.3
(22) Date of filing: 20.02.2014
(51) Int. Cl.: H01L 51/54, C09K 11/06, C07D 405/04, C07D 409/04

(54) **COMPOSITION AND ORGANIC OPTOELECTRONIC ELEMENT**
ZUSAMMENSETZUNG UND ORGANISCHES OPTOELEKTRONISCHES ELEMENT
COMPOSITION ET ELÉMENT OPTOÉLECTRONIQUE ORGANIQUE

(30) Priority: 12.08.2013 KR 20130095619
(43) Date of publication of application: 22.06.2016
(73) Proprietor: Samsung SDI Co., Ltd., Gyeonggi-do 17084 (KR)
(72) Inventor: JO, Young-Kyoung, Uiwang-si Gyeonggi-do 437-711 (KR); LEE, Han-Ill, Uiwang-si Gyeonggi-do 437-711 (KR); KANG, Eui-Su, Uiwang-si Gyeonggi-do 437-711 (KR); KIM, Youn-Hwan, Uiwang-si Gyeonggi-do 437-711 (KR); YANG, Yong-Tak, Uiwang-si Gyeonggi-do 437-711 (KR); OH, Jae-Jin, Uiwang-si Gyeonggi-do 437-711 (KR); LEE, Nam-Heon, Uiwang-si Gyeonggi-do 437-711 (KR); LUI, Jin-Hyun, Uiwang-si Gyeonggi-do 437-711 (KR); MIN, Soo-Hyun, Uiwang-si Gyeonggi-do 437-711 (KR); YU, Eun-Sun, Uiwang-si Gyeonggi-do 437-711 (KR); JUNG, Ho-Kuk, Uiwang-si Gyeonggi-do 437-711 (KR)
(74) Representative: Michalski Hüttermann & Partner Patentanwälte mbB
(86) International application number: PCT/KR2014/001390
(87) International publication number: WO 2015/023034

(56) References cited:
- EP-A1- 2 403 028
- WO-A1-2011/137072
- WO-A1-2012/023947
- WO-A1-2012/153725
- WO-A1-2013/094854
- WO-A1-2013/109045
- WO-A2-2009/021126
- WO-A2-2011/055933
- SOON OK JEON ET AL: "Fluorenobenzofuran as the core structure of high triplet energy host materials for green phosphorescent organic light-emitting diodes", JOURNAL OF MATERIALS CHEMISTRY, vol. 22, no. 21, 20 March 2012 (2012-03-20), page 10537, XP055198769, ISSN: 0959-9428, DOI: 10.1039/c2jm30473b

## Description

### [Technical Field]

A composition, an organic optoelectronic device and a display device are disclosed.

### [Background Art]

An organic optoelectronic device is a device that converts electrical energy into photoenergy, and vice versa.

An organic optoelectronic device may be classified as follows in accordance with its driving principles. One is an optoelectronic device where excitons are generated by photoenergy, separated into electrons and holes, and are transferred to different electrodes to generate electrical energy, and the other is a light emitting device where a voltage or a current is supplied to an electrode to generate photoenergy from electrical energy.

Examples of the organic optoelectronic device may be an organic photoelectric device, an organic light emitting diode, an organic solar cell, and an organic photo conductor drum.

Of these, an organic light emitting diode (OLED) has recently drawn attention due to an increase in demand for flat panel displays. Such an organic light emitting diode converts electrical energy into light by applying current to an organic light emitting material. It has a structure in which an organic layer is interposed between an anode and a cathode. Herein, the organic layer may include an emission layer and optionally an auxiliary layer, and the auxiliary layer may be, for example at least one selected from a hole injection layer, a hole transport layer, an electron blocking layer, an electron transport layer, an electron injection layer, and a hole blocking layer for improving efficiency and stability of an organic light emitting diode.

Performance of an organic light emitting diode may be affected by characteristics of the organic layer, and among them, may be mainly affected by characteristics of an organic material of the organic layer.

Particularly, development for an organic material being capable of increasing hole and electron mobility and simultaneously increasing electrochemical stability is needed so that the organic light emitting diode may be applied to a large-size flat panel display.

WO 2013/109045 A1, EP 2 403 028 A1 and EP 2 709 181 A1 disclose organic electroluminescent devices and compounds for such devices.

J. Mater. Chem.' 2012' 22' 10537-10541 discloses fluorobenzofuran derivatives and their use in organic electroluminescent devices.

### [Disclosure]

### [Technical object]

One embodiment provides a composition for an organic optoelectronic device being capable of realizing an organic optoelectronic device having high efficiency and long life-span.

Another embodiment provides an organic optoelectronic device including the composition.

Yet another embodiment provides a display device including the organic optoelectronic device.

### [Technical Solving Method]

According to one embodiment, a composition includes a first host compound represented by Chemical Formula I and a second host compound represented by Chemical Formula II. 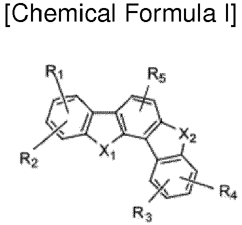
In Chemical Formula I,
X₁ is O, Se, S, SO, SO₂, PO, or CO,
X₂ is CRₐR_{b} or SiRcR_{d}, and
R₁ to R₅ and Rₐ to R_{d} are each independently hydrogen, deuterium, a substituted or unsubstituted C1 to C30 alkyl group, a substituted or unsubstituted C3 to C30 cycloalkyl group, a substituted or unsubstituted C3 to C30 heterocycloalkyl group, a substituted or unsubstituted C6 to C30 aryl group, a substituted or unsubstituted C3 to C30 heteroaryl group, a substituted or unsubstituted amine group, a substituted or unsubstituted C6 to C30 arylamine group, a substituted or unsubstituted C3 to C30 heteroarylamine group, a substituted or unsubstituted C1 to C30 alkoxy group, a substituted or unsubstituted C2 to C30 alkoxycarbonyl group, a substituted or unsubstituted C2 to C30 alkoxycarbonylamino group, a substituted or unsubstituted C7 to C30 aryloxycarbonylamino group, a substituted or unsubstituted C1 to C30 sulfamoylamino group, a substituted or unsubstituted C2 to C30 alkenyl group, a substituted or unsubstituted C2 to C30 alkynyl group, a substituted or unsubstituted silyl group, a substituted or unsubstituted silyloxy group, a substituted or unsubstituted C1 to C30 acyl group, a substituted or unsubstituted C1 to C20 acyloxy group, a substituted or unsubstituted C1 to C20 acylamino group, a substituted or unsubstituted C1 to C30 sulfonyl group, a substituted or unsubstituted C1 to C30 alkylthiol group, a substituted or unsubstituted C1 to C30 heterocyclothiol group, a substituted or unsubstituted C6 to C30 arylthiol group, a substituted or unsubstituted C1 to C30 heteroarylthiol group, a substituted or unsubstituted C1 to C30 ureide group, a halogen, a halogen-containing group, a cyano group, a hydroxyl group, an amino group, a nitro group, a carboxyl group, a ferrocenyl group, or a combination thereof. 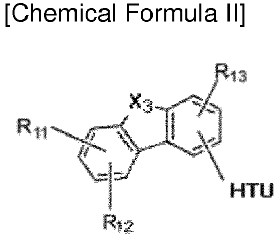
In Chemical Formula II,
X₃ is O, S, CRᵤRᵥ, SiR_{w}Rₓ, or NR_{y},
HTU is a substituted or unsubstituted carbazolyl group or a substituted or unsubstituted triphenylenyl group, and
R₁₁ to R₁₃ and Rᵤ to R_{y} are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.

According to another embodiment, an organic optoelectronic device includes an anode and a cathode facing each other, at least one organic layer between the anode and the cathode, wherein the organic layer includes the composition.

According to yet another embodiment, a display device including the organic optoelectronic device is provided.

### [Advantageous Effect]

An organic optoelectronic device having high efficiency and long life-span may be realized.

### [Description of Drawings]

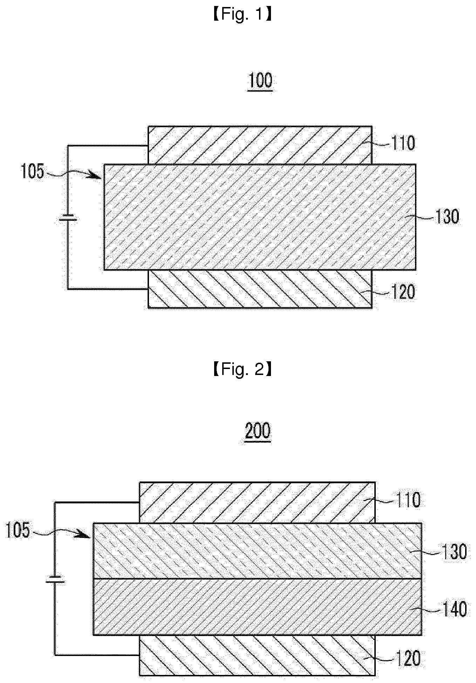

FIGS. 1 and 2 are cross-sectional views showing organic light emitting diodes according to one embodiment, respectively.

### [Best Mode]

Hereinafter, embodiments of the present invention are described in detail. However, these embodiments are exemplary, the present invention is not limited thereto and the present invention is defined by the scope of claims.

In the present specification, when a definition is not otherwise provided, the term "substituted" refers to one substituted with a deuterium, a halogen, a hydroxy group, an amino group, a substituted or unsubstituted C1 to C30 amine group, a nitro group, a substituted or unsubstituted C1 to C40 silyl group, C1 to C30 alkyl group, a C1 to C10 alkylsilyl group, a C3 to C30 cycloalkyl group, a C3 to C30 heterocycloalkyl group, a C6 to C30 aryl group, a C6 to C30 heteroaryl group, a C1 to C20 alkoxy group, a fluoro group, a C1 to C10 trifluoroalkyl group such as a trifluoromethyl group, or a cyano group, instead of at least one hydrogen of a substituent or a compound.

In addition, two adjacent substituents of the halogen, hydroxy group, amino group, substituted or unsubstituted C1 to C20 amine group, nitro group, substituted or unsubstituted C3 to C40 silyl group, C1 to C30 alkyl group, C1 to C10 alkylsilyl group, C3 to C30 cycloalkyl group, C3 to C30 heterocycloalkyl group, C6 to C30 aryl group, C6 to C30 heteroaryl group, C1 to C20 alkoxy group, fluoro group, C1 to C10 trifluoroalkyl group such as trifluoromethyl group and the like, or cyano group may be fused with each other to form a ring. For example, the substituted C6 to C30 aryl group may be fused with another adjacent substituted C6 to C30 aryl group to form a substituted or unsubstituted fluorene ring.

In the present specification, when specific definition is not otherwise provided, "hetero" refers to one including 1 to 3 hetero atoms selected from the group consisting of N, O, S, P, and Si, and remaining carbons in one compound or substituent.

In the present specification, when a definition is not otherwise provided, "alkyl group" refers to an aliphatic hydrocarbon group. The alkyl group may be "a saturated alkyl group" without any double bond or triple bond.

The alkyl group may be a C1 to C30 alkyl group. More specifically, the alkyl group may be a C1 to C20 alkyl group or a C1 to C10 alkyl group. For example, a C1 to C4 alkyl group may have 1 to 4 carbon atoms in an alkyl chain which may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a t-butyl group, a pentyl group, a hexyl group, a cyclopropyl group, a cyclobutyl group, a cyclopentyl group, a cyclohexyl group, and the like.

In the present specification, "aryl group" refers to a cyclic substituent where all elements have p-orbitals, and these p-orbitals forms conjugation, and includes a monocyclic, polycyclic or fused ring polycyclic (i.e., rings sharing adjacent pairs of carbon atoms) functional group.

In the present specification, "heteroaryl group" refers to aryl group including 1 to 3 hetero atoms selected from N, O, S, P and Si, and remaining carbons. When the heteroaryl group is a fused ring, each ring may include 1 to 3 hetero atoms.

More specifically, the substituted or unsubstituted C6 to C30 aryl group and/or the substituted or unsubstituted C2 to C30 heteroaryl group refer to a substituted or unsubstituted phenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted chrysenyl group, a substituted or unsubstituted triphenylenyl group, a substituted or unsubstituted perylenyl group, a substituted or unsubstituted indenyl group, a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted fluorenyl group, a substituted or unsubstituted dibenzofuranyl group, a substituted or unsubstituted dibenzothiophenyl group, a substituted or unsubstituted carbazolyl group, or a combination thereof, but is not limited thereto.

In the present specification, hole characteristics refer to characteristics to donate an electron and to form a hole when an electric field is applied, and characteristics that holes formed in the anode is easily injected into the emission layer and transported in the emission layer due to conductive characteristics according to HOMO level.

Electron characteristics refer to characteristics to accept an electron when an electric field is applied, and characteristics that electron formed in the cathode is easily injected into the emission layer and transported in the emission layer due to conductive characteristics according to LUMO level.

Hereinafter, a composition according to one embodiment is described.

The composition according to one embodiment includes at least two kinds of hosts and dopant, wherein the hosts include a first host compound having bipolar characteristics and a second host compound having hole characteristics and at least two fused rings.

The first host compound is represented by Chemical Formula I. 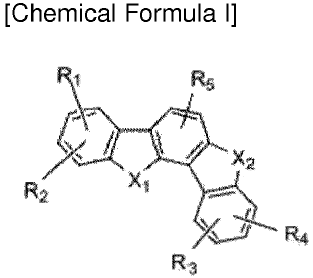
In Chemical Formula I,
X₁ is O, Se, S, SO, SO₂, PO, or CO,
X₂ is CRₐR_{b} or SiR_{c}R_{d}, and
R₁ to R₅ and Rₐ to R_{d} are each independently hydrogen, deuterium, a substituted or unsubstituted C1 to C30 alkyl group, a substituted or unsubstituted C3 to C30 cycloalkyl group, a substituted or unsubstituted C3 to C30 heterocycloalkyl group, a substituted or unsubstituted C6 to C30 aryl group, a substituted or unsubstituted C3 to C30 heteroaryl group, a substituted or unsubstituted amine group, a substituted or unsubstituted C6 to C30 arylamine group, a substituted or unsubstituted C3 to C30 heteroarylamine group, a substituted or unsubstituted C1 to C30 alkoxy group, a substituted or unsubstituted C2 to C30 alkoxycarbonyl group, a substituted or unsubstituted C2 to C30 alkoxycarbonylamino group, a substituted or unsubstituted C7 to C30 aryloxycarbonylamino group, a substituted or unsubstituted C1 to C30 sulfamoylamino group, a substituted or unsubstituted C2 to C30 alkenyl group, a substituted or unsubstituted C2 to C30 alkynyl group, a substituted or unsubstituted silyl group, a substituted or unsubstituted silyloxy group, a substituted or unsubstituted C1 to C30 acyl group, a substituted or unsubstituted C1 to C20 acyloxy group, a substituted or unsubstituted C1 to C20 acylamino group, a substituted or unsubstituted C1 to C30 sulfonyl group, a substituted or unsubstituted C1 to C30 alkylthiol group, a substituted or unsubstituted C1 to C30 heterocyclothiol group, a substituted or unsubstituted C6 to C30 arylthiol group, a substituted or unsubstituted C1 to C30 heteroarylthiol group, a substituted or unsubstituted C1 to C30 ureide group, a halogen, a halogen-containing group, a cyano group, a hydroxyl group, an amino group, a nitro group, a carboxyl group, a ferrocenyl group or a combination thereof.

The first host compound may be for example represented by one of Chemical Formulae 1a to 1c according to a bonding position of a substituent.

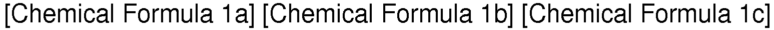

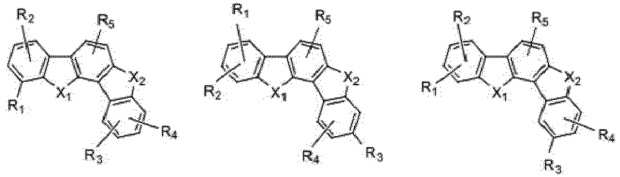

In Chemical Formulae 1a to 1c, X₁, X₂, R₁ to R₅ and Rₐ to R_{d} are the same as described above.

The first host compound may be for example represented by one of Chemical Formulae 1-1 to 1-6. 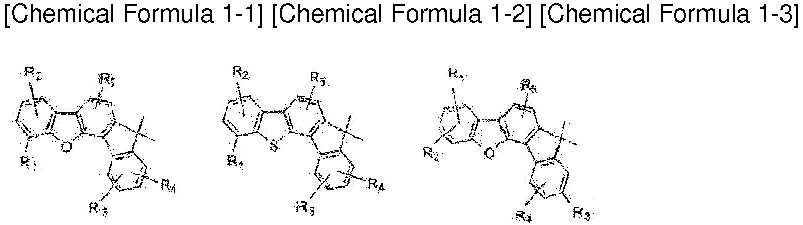 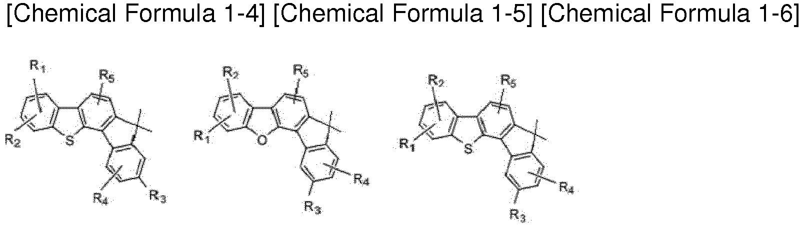
In Chemical Formulae 1-1 to 1-6,
R₁ to R₅ are the same as described above, for example R₁ to R₅ are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.
At least one of R₁ to R₅ and Rₐ to R_{d} of In Chemical Formula I, Chemical Formulae 1a to 1c and Chemical Formulae 1-1 to 1-6 may be a substituent having hole characteristics or electron characteristics, and may be, for example selected from a substituted or unsubstituted functional group listed in Group 1. 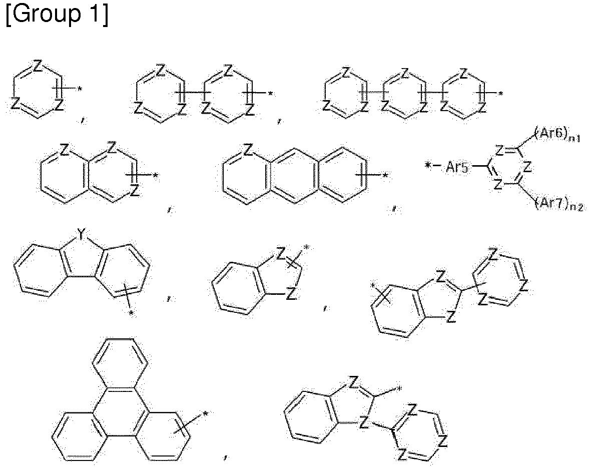
In Group 1,
Y is N, O, S, SO₂, NRⱼ, CRₖ, SiRₗ, CRₘRₙ or SiRₒRₚ,
Z is N, CR_{q}, CRᵣRₛ, or NRₜ,
wherein Rⱼ to Rₜ are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof,
Ar5 is a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, a substituted or unsubstituted C6 to C30 arylamine group, a substituted or unsubstituted C2 to C30 heteroarylamine group, or a combination thereof,
Ar6 and Ar7 are independently substituted or unsubstituted C6 to C30 aryl group, a substituted or unsubstituted C3 to C30 heteroaryl group, or a combination thereof,
n1 and n2 are each independently 0 or 1, and
* is a linking point to Chemical Formula 1 and may be positioned at one of elements of the functional group.

The substituted or unsubstituted functional group of Group 1 may be, for example selected from Chemical Formulae B-1 to B-35.

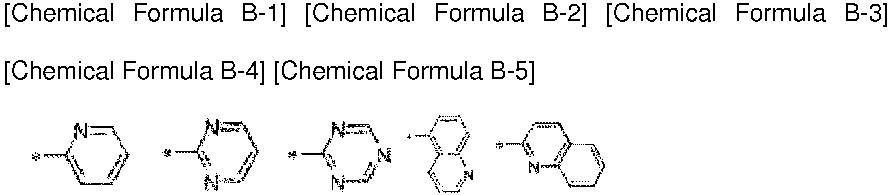

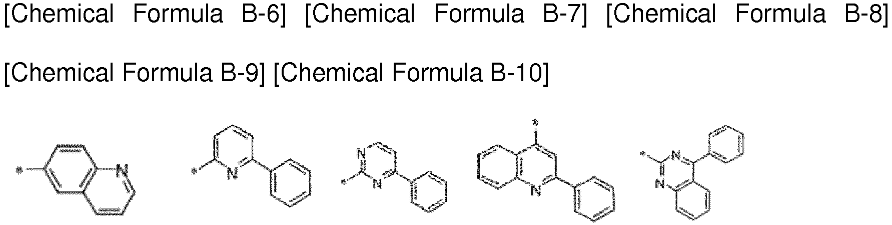

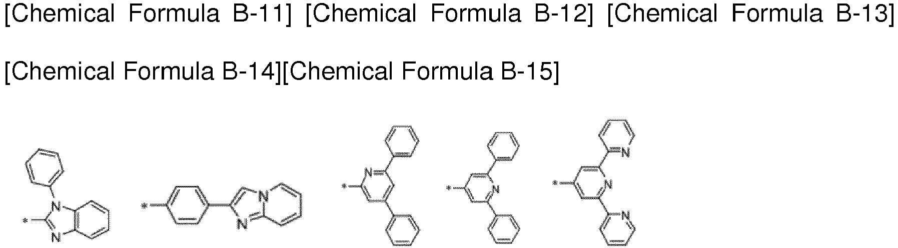

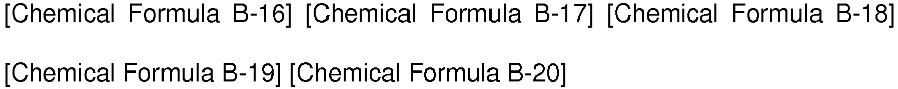

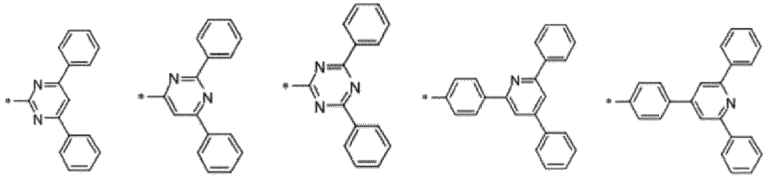

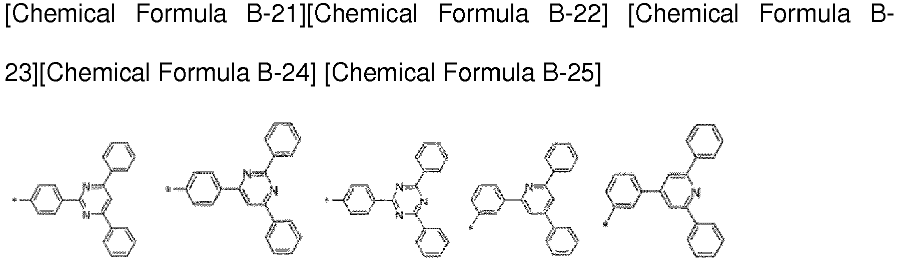

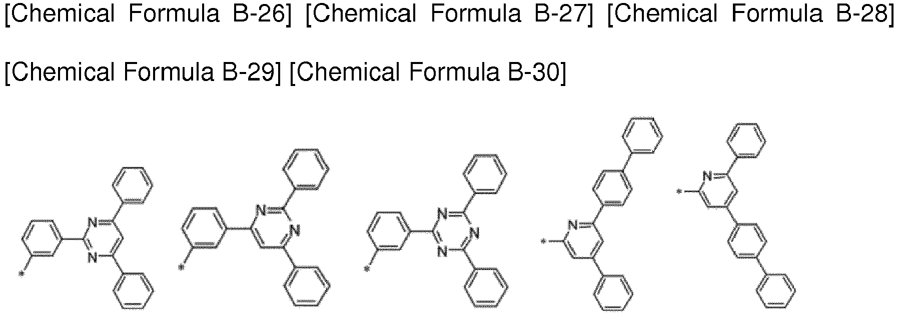

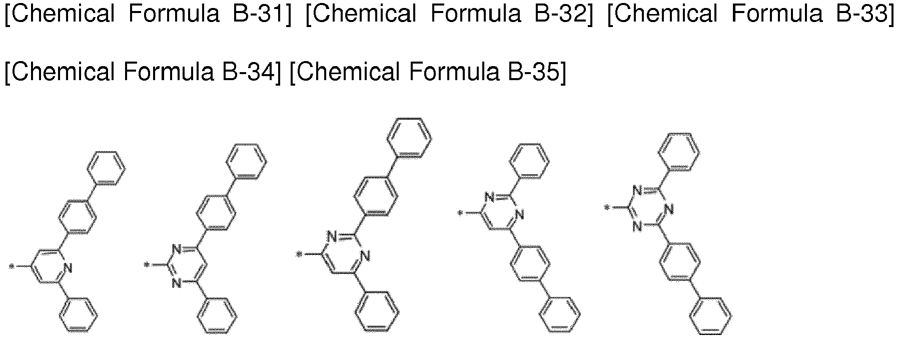

The first host compound may be for example represented in [Table 1] and [Table 2], and herein, the substituent indicates R₁ when a core moiety is 1-1 and 1-2 and the substituent indicates R₃ when the core moiety is 1-3 to 1-6, and particularly, other non-mentioned substituents are all hydrogen.

**(Table 1)**

| Compound Nos. | Core moiety | substituent | Compound Nos. | Core moiety | Substituent | Compound Nos. | Core moiety | Substituent |
|---|---|---|---|---|---|---|---|---|
| 1 | 1-1 | B-1 | 41 | 1-2 | B-6 | 81 | 1-3 | B-11 |
| 2 | 1-1 | B-2 | 42 | 1-2 | B-7 | 82 | 1-3 | B-12 |
| 3 | 1-1 | B-3 | 43 | 1-2 | B-8 | 83 | 1-3 | B-13 |
| 4 | 1-1 | B-4 | 44 | 1-2 | B-9 | 84 | 1-3 | B-14 |
| 5 | 1-1 | B-5 | 45 | 1-2 | B-10 | 85 | 1-3 | B-15 |
| 6 | 1-1 | B-6 | 46 | 1-2 | B-11 | 86 | 1-3 | B-16 |
| 7 | 1-1 | B-7 | 47 | 1-2 | B-12 | 87 | 1-3 | B-17 |
| 8 | 1-1 | B-8 | 48 | 1-2 | B-13 | 88 | 1-3 | B-18 |
| 9 | 1-1 | B-9 | 49 | 1-2 | B-14 | 89 | 1-3 | B-19 |
| 10 | 1-1 | B-10 | 50 | 1-2 | B-15 | 90 | 1-3 | B-20 |
| 11 | 1-1 | B-11 | 51 | 1-2 | B-16 | 91 | 1-3 | B-21 |
| 12 | 1-1 | B-12 | 52 | 1-2 | B-17 | 92 | 1-3 | B-22 |
| 13 | 1-1 | B-13 | 53 | 1-2 | B-18 | 93 | 1-3 | B-23 |
| 14 | 1-1 | B-14 | 54 | 1-2 | B-19 | 94 | 1-3 | B-24 |
| 15 | 1-1 | B-15 | 55 | 1-2 | B-20 | 95 | 1-3 | B-25 |
| 16 | 1-1 | B-16 | 56 | 1-2 | B-21 | 96 | 1-3 | B-26 |
| 17 | 1-1 | B-17 | 57 | 1-2 | B-22 | 97 | 1-3 | B-27 |
| 18 | 1-1 | B-18 | 58 | 1-2 | B-23 | 98 | 1-3 | B-28 |
| 19 | 1-1 | B-19 | 59 | 1-2 | B-24 | 99 | 1-3 | B-29 |
| 20 | 1-1 | B-20 | 60 | 1-2 | B-25 | 100 | 1-3 | B-30 |
| 21 | 1-1 | B-21 | 61 | 1-2 | B-26 | 101 | 1-3 | B-31 |
| 22 | 1-1 | B-22 | 62 | 1-2 | B-27 | 102 | 1-3 | B-32 |
| 23 | 1-1 | B-23 | 63 | 1-2 | B-28 | 103 | 1-3 | B-33 |
| 24 | 1-1 | B-24 | 64 | 1-2 | B-29 | 104 | 1-3 | B-34 |
| 25 | 1-1 | B-25 | 65 | 1-2 | B-30 | 105 | 1-3 | B-35 |
| 26 | 1-1 | B-26 | 66 | 1-2 | B-31 | 106 | 1-4 | B-1 |
| 27 | 1-1 | B-27 | 67 | 1-2 | B-32 | 107 | 1-4 | B-2 |
| 28 | 1-1 | B-28 | 68 | 1-2 | B-33 | 108 | 1-4 | B-3 |
| 29 | 1-1 | B-29 | 69 | 1-2 | B-34 | 109 | 1-4 | B-4 |
| 30 | 1-1 | B-30 | 70 | 1-2 | B-35 | 110 | 1-4 | B-5 |
| 31 | 1-1 | B-31 | 71 | 1-3 | B-1 | 111 | 1-4 | B-6 |
| 32 | 1-1 | B-32 | 72 | 1-3 | B-2 | 112 | 1-4 | B-7 |
| 33 | 1-1 | B-33 | 73 | 1-3 | B-3 | 113 | 1-4 | B-8 |
| 34 | 1-1 | B-34 | 74 | 1-3 | B-4 | 114 | 1-4 | B-9 |
| 35 | 1-1 | B-35 | 75 | 1-3 | B-5 | 115 | 1-4 | B-10 |
| 36 | 1-2 | B-1 | 76 | 1-3 | B-6 | 116 | 1-4 | B-11 |
| 37 | 1-2 | B-2 | 77 | 1-3 | B-7 | 117 | 1-4 | B-12 |
| 38 | 1-2 | B-3 | 78 | 1-3 | B-8 | 118 | 1-4 | B-13 |
| 39 | 1-2 | B-4 | 79 | 1-3 | B-9 | 119 | 1-4 | B-14 |
| 40 | 1-2 | B-5 | 80 | 1-3 | B-10 | 120 | 1-4 | B-15 |

**(Table 2)**

| Compound Nos. | Core | Substituent | Compound Nos. | Core | Substituent |
|---|---|---|---|---|---|
| 121 | 1-4 | B-16 | 161 | 1-5 | B-21 |
| 122 | 1-4 | B-17 | 162 | 1-5 | B-22 |
| 123 | 1-4 | B-18 | 163 | 1-5 | B-23 |
| 124 | 1-4 | B-19 | 164 | 1-5 | B-24 |
| 125 | 1-4 | B-20 | 165 | 1-5 | B-25 |
| 126 | 1-4 | B-21 | 166 | 1-5 | B-26 |
| 127 | 1-4 | B-22 | 167 | 1-5 | B-27 |
| 128 | 1-4 | B-23 | 168 | 1-5 | B-28 |
| 129 | 1-4 | B-24 | 169 | 1-5 | B-29 |
| 130 | 1-4 | B-25 | 170 | 1-5 | B-30 |
| 131 | 1-4 | B-26 | 171 | 1-5 | B-31 |
| 132 | 1-4 | B-27 | 172 | 1-5 | B-32 |
| 133 | 1-4 | B-28 | 173 | 1-5 | B-33 |
| 134 | 1-4 | B-29 | 174 | 1-5 | B-34 |
| 135 | 1-4 | B-30 | 175 | 1-5 | B-35 |
| 136 | 1-4 | B-31 | 176 | 1-6 | B-1 |
| 137 | 1-4 | B-32 | 177 | 1-6 | B-2 |
| 138 | 1-4 | B-33 | 178 | 1-6 | B-3 |
| 139 | 1-4 | B-34 | 179 | 1-6 | B-4 |
| 140 | 1-4 | B-35 | 180 | 1-6 | B-5 |
| 141 | 1-5 | B-1 | 181 | 1-6 | B-6 |
| 142 | 1-5 | B-2 | 182 | 1-6 | B-7 |
| 143 | 1-5 | B-3 | 183 | 1-6 | B-8 |
| 144 | 1-5 | B-4 | 184 | 1-6 | B-9 |
| 145 | 1-5 | B-5 | 185 | 1-6 | B-10 |
| 146 | 1-5 | B-6 | 186 | 1-6 | B-11 |
| 147 | 1-5 | B-7 | 187 | 1-6 | B-12 |
| 148 | 1-5 | B-8 | 188 | 1-6 | B-13 |
| 149 | 1-5 | B-9 | 189 | 1-6 | B-14 |
| 150 | 1-5 | B-10 | 190 | 1-6 | B-15 |
| 151 | 1-5 | B-11 | 191 | 1-6 | B-16 |
| 152 | 1-5 | B-12 | 192 | 1-6 | B-17 |
| 153 | 1-5 | B-13 | 193 | 1-6 | B-18 |
| 154 | 1-5 | B-14 | 194 | 1-6 | B-19 |
| 155 | 1-5 | B-15 | 195 | 1-6 | B-20 |
| 156 | 1-5 | B-16 | 196 | 1-6 | B-21 |
| 157 | 1-5 | B-17 | 197 | 1-6 | B-22 |
| 158 | 1-5 | B-18 | 198 | 1-6 | B-23 |
| 159 | 1-5 | B-19 | 199 | 1-6 | B-24 |
| 160 | 1-5 | B-20 | 200 | 1-6 | B-25 |

The first host compound may be, for example, represented by one of Chemical Formulae 1-A to 1-P.

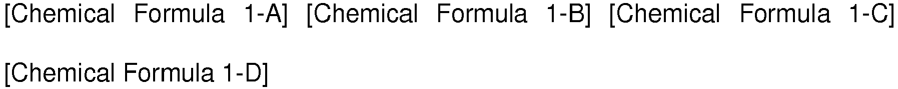

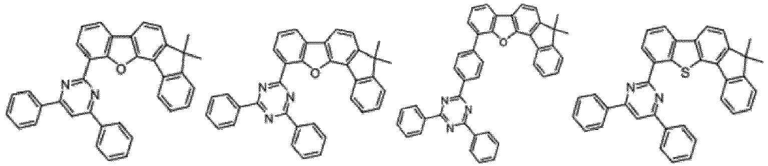

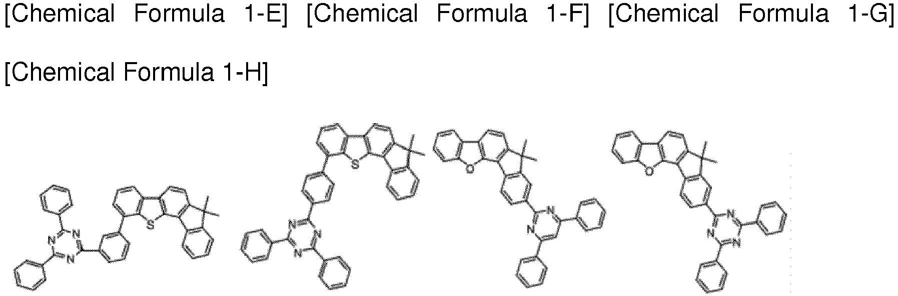

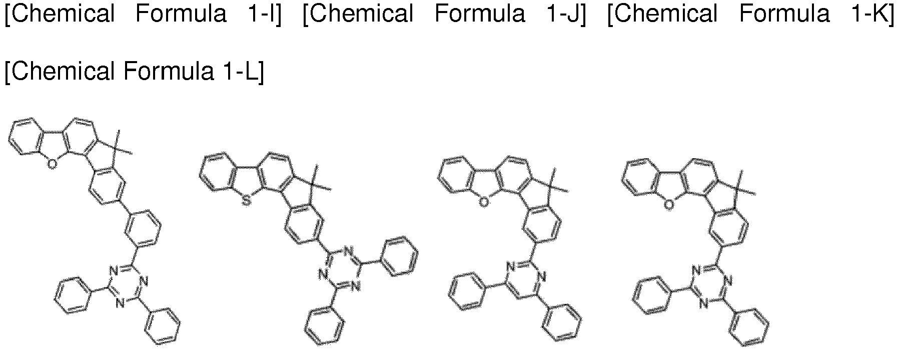

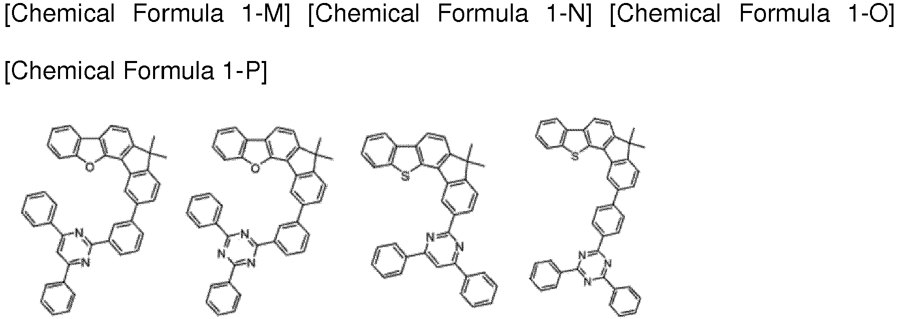

The second host compound may be represented by Chemical Formula II. 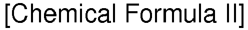 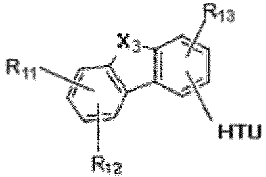
In Chemical Formula II,
X₃ is O, S, CRᵤRᵥ, SiR_{w}Rₓ, or NR_{y},
HTU is a substituted or unsubstituted carbazolyl group or a substituted or unsubstituted triphenylenyl group, and
R₁₁ to R₁₃ and Rᵤ to R_{y} are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.

The second host compound may be, for example a compound including two carbazole groups represented by Chemical Formula 2. 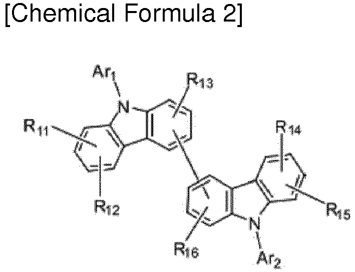
In Chemical Formula 2,
Ar₁, Ar₂, and R₁₁ to R₁₆ are the same as R₁ to R₅, for example Ar₁, Ar₂ and R₁₁ to R₁₆ are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.

The compound represented by Chemical Formula 2 may be for example represented by one of Chemical Formulae 2-1 to 2-3 according to a bonding position of a substituent.

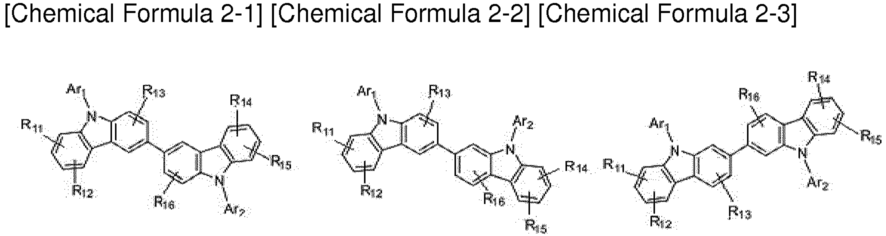

In Chemical Formulae 2-1 to 2-3, Ar₁, Ar₂, and R₁₁ to R₁₆ are the same as described above.

At least one of Ar₁, Ar₂, and R₁₁ to R₁₆ of Chemical Formula 2 and Chemical Formulae 2-1 to 2-3 may be a substituent having hole characteristics, for example a substituent selected from Chemical Formulae A-1 to A-15. [Chemical Formula A-1] [Chemical Formula A-2][Chemical Formula A-

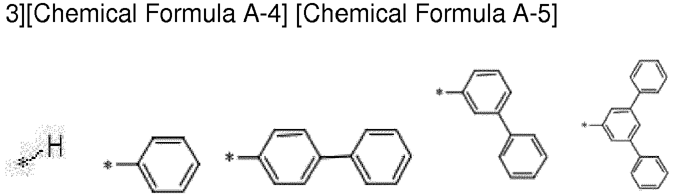

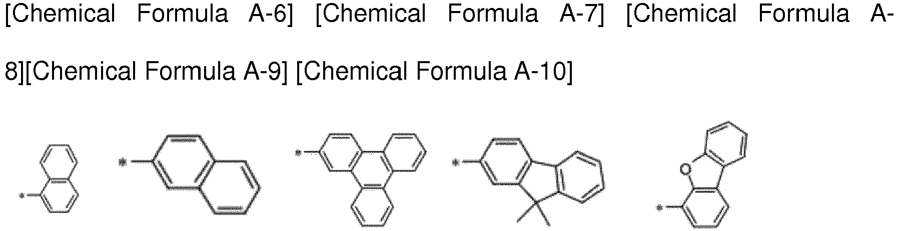

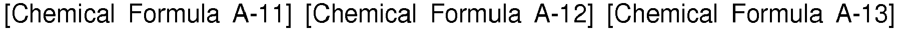

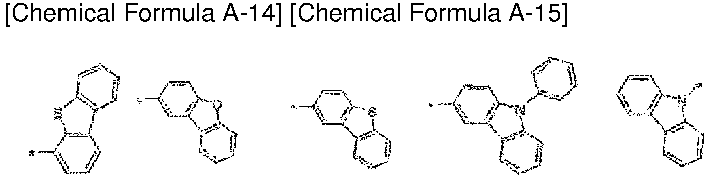

The compound represented by Chemical Formula 2 may be, for example represented in [Table 3] to [Table 5], wherein substituents that is not mentioned, R₁₁ to R₁₆ are all hydrogen.

**(Table 3)**

| Comp -ound Nos. | Core | Substit -uent Ar1 | Substit -uent Ar2 | Comp -ound Nos. | Core | Substit -uent Ar1 | Substit -uent Ar2 | Comp -ound Nos. | Core | Substit -uent Ar1 | Substit -uent Ar2 |
|---|---|---|---|---|---|---|---|---|---|---|---|
| 201 | 2-1 | A-2 | A-2 | 241 | 2-1 | A-5 | A-6 | 281 | 2-1 | A-9 | A-12 |
| 202 | 2-1 | A-2 | A-3 | 242 | 2-1 | A-5 | A-7 | 282 | 2-1 | A-9 | A-13 |
| 203 | 2-1 | A-2 | A-4 | 243 | 2-1 | A-5 | A-8 | 283 | 2-1 | A-9 | A-14 |
| 204 | 2-1 | A-2 | A-5 | 244 | 2-1 | A-5 | A-9 | 284 | 2-1 | A-9 | A-15 |
| 205 | 2-1 | A-2 | A-6 | 245 | 2-1 | A-5 | A-10 | 285 | 2-1 | A-10 | A-10 |
| 206 | 2-1 | A-2 | A-7 | 246 | 2-1 | A-5 | A-11 | 286 | 2-1 | A-10 | A-11 |
| 207 | 2-1 | A-2 | A-8 | 247 | 2-1 | A-5 | A-12 | 287 | 2-1 | A-10 | A-12 |
| 208 | 2-1 | A-2 | A-9 | 248 | 2-1 | A-5 | A-13 | 288 | 2-1 | A-10 | A-13 |
| 209 | 2-1 | A-2 | A-10 | 249 | 2-1 | A-5 | A-14 | 289 | 2-1 | A-10 | A-14 |
| 210 | 2-1 | A-2 | A-11 | 250 | 2-1 | A-5 | A-15 | 290 | 2-1 | A-10 | A-15 |
| 211 | 2-1 | A-2 | A-12 | 251 | 2-1 | A-6 | A-6 | 291 | 2-1 | A-11 | A-11 |
| 212 | 2-1 | A-2 | A-13 | 252 | 2-1 | A-6 | A-7 | 292 | 2-1 | A-11 | A-12 |
| 213 | 2-1 | A-2 | A-14 | 253 | 2-1 | A-6 | A-8 | 293 | 2-1 | A-11 | A-13 |
| 214 | 2-1 | A-2 | A-15 | 254 | 2-1 | A-6 | A-9 | 294 | 2-1 | A-11 | A-14 |
| 215 | 2-1 | A-3 | A-3 | 255 | 2-1 | A-6 | A-10 | 295 | 2-1 | A-11 | A-15 |
| 216 | 2-1 | A-3 | A-4 | 256 | 2-1 | A-6 | A-11 | 296 | 2-1 | A-12 | A-12 |
| 217 | 2-1 | A-3 | A-5 | 257 | 2-1 | A-6 | A-12 | 297 | 2-1 | A-12 | A-13 |
| 218 | 2-1 | A-3 | A-6 | 258 | 2-1 | A-6 | A-13 | 298 | 2-1 | A-12 | A-14 |
| 219 | 2-1 | A-3 | A-7 | 259 | 2-1 | A-6 | A-14 | 299 | 2-1 | A-12 | A-15 |
| 220 | 2-1 | A-3 | A-8 | 260 | 2-1 | A-6 | A-15 | 300 | 2-1 | A-13 | A-13 |
| 221 | 2-1 | A-3 | A-9 | 261 | 2-1 | A-7 | A-7 | 301 | 2-1 | A-13 | A-14 |
| 222 | 2-1 | A-3 | A-10 | 262 | 2-1 | A-7 | A-8 | 302 | 2-1 | A-13 | A-15 |
| 223 | 2-1 | A-3 | A-11 | 263 | 2-1 | A-7 | A-9 | 303 | 2-1 | A-14 | A-14 |
| 224 | 2-1 | A-3 | A-12 | 264 | 2-1 | A-7 | A-10 | 304 | 2-1 | A-14 | A-15 |
| 225 | 2-1 | A-3 | A-13 | 265 | 2-1 | A-7 | A-11 | 305 | 2-1 | A-15 | A-15 |
| 226 | 2-1 | A-3 | A-14 | 266 | 2-1 | A-7 | A-12 | 306 | 2-2 | A-2 | A-2 |
| 227 | 2-1 | A-3 | A-15 | 267 | 2-1 | A-7 | A-13 | 307 | 2-2 | A-2 | A-3 |
| 228 | 2-1 | A-4 | A-4 | 268 | 2-1 | A-7 | A-14 | 308 | 2-2 | A-2 | A-4 |
| 229 | 2-1 | A-4 | A-5 | 269 | 2-1 | A-7 | A-15 | 309 | 2-2 | A-2 | A-5 |
| 230 | 2-1 | A-4 | A-6 | 270 | 2-1 | A-8 | A-8 | 310 | 2-2 | A-2 | A-6 |
| 231 | 2-1 | A-4 | A-7 | 271 | 2-1 | A-8 | A-9 | 311 | 2-2 | A-2 | A-7 |
| 232 | 2-1 | A-4 | A-8 | 272 | 2-1 | A-8 | A-10 | 312 | 2-2 | A-2 | A-8 |
| 233 | 2-1 | A-4 | A-9 | 273 | 2-1 | A-8 | A-11 | 313 | 2-2 | A-2 | A-9 |
| 234 | 2-1 | A-4 | A-10 | 274 | 2-1 | A-8 | A-12 | 314 | 2-2 | A-2 | A-10 |
| 235 | 2-1 | A-4 | A-11 | 275 | 2-1 | A-8 | A-13 | 315 | 2-2 | A-2 | A-11 |
| 236 | 2-1 | A-4 | A-12 | 276 | 2-1 | A-8 | A-14 | 316 | 2-2 | A-2 | A-12 |
| 237 | 2-1 | A-4 | A-13 | 277 | 2-1 | A-8 | A-15 | 317 | 2-2 | A-2 | A-13 |
| 238 | 2-1 | A-4 | A-14 | 278 | 2-1 | A-9 | A-9 | 318 | 2-2 | A-2 | A-14 |
| 239 | 2-1 | A-4 | A-15 | 279 | 2-1 | A-9 | A-10 | 319 | 2-2 | A-2 | A-15 |
| 240 | 2-1 | A-5 | A-5 | 280 | 2-1 | A-9 | A-11 | 320 | 2-2 | A-3 | A-3 |

**(Table 4)**

| Compo -und Nos. | Co -re | Substituent Ar1 | Substituent Ar2 | Compo -und Nos. | Co -re | Substituent Ar1 | Substituent Ar2 | Compo -und Nos. | Core | Substi -tuent Ar1 | Substituent Ar2 |
|---|---|---|---|---|---|---|---|---|---|---|---|
| 321 | 2-2 | A-3 | A-4 | 361 | 2-2 | A-6 | A-11 | 401 | 2-2 | A-12 | A-12 |
| 322 | 2-2 | A-3 | A-5 | 362 | 2-2 | A-6 | A-12 | 402 | 2-2 | A-12 | A-13 |
| 323 | 2-2 | A-3 | A-6 | 363 | 2-2 | A-6 | A-13 | 403 | 2-2 | A-12 | A-14 |
| 324 | 2-2 | A-3 | A-7 | 364 | 2-2 | A-6 | A-14 | 404 | 2-2 | A-12 | A-15 |
| 325 | 2-2 | A-3 | A-8 | 365 | 2-2 | A-6 | A-15 | 405 | 2-2 | A-13 | A-13 |
| 326 | 2-2 | A-3 | A-9 | 366 | 2-2 | A-7 | A-7 | 406 | 2-2 | A-13 | A-14 |
| 327 | 2-2 | A-3 | A-10 | 367 | 2-2 | A-7 | A-8 | 407 | 2-2 | A-13 | A-15 |
| 328 | 2-2 | A-3 | A-11 | 368 | 2-2 | A-7 | A-9 | 408 | 2-2 | A-14 | A-14 |
| 329 | 2-2 | A-3 | A-12 | 369 | 2-2 | A-7 | A-10 | 409 | 2-2 | A-14 | A-15 |
| 330 | 2-2 | A-3 | A-13 | 370 | 2-2 | A-7 | A-11 | 410 | 2-2 | A-15 | A-15 |
| 331 | 2-2 | A-3 | A-14 | 371 | 2-2 | A-7 | A-12 | 411 | 2-3 | A-2 | A-2 |
| 332 | 2-2 | A-3 | A-15 | 372 | 2-2 | A-7 | A-13 | 412 | 2-3 | A-2 | A-3 |
| 333 | 2-2 | A-4 | A-4 | 373 | 2-2 | A-7 | A-14 | 413 | 2-3 | A-2 | A-4 |
| 334 | 2-2 | A-4 | A-5 | 374 | 2-2 | A-7 | A-15 | 414 | 2-3 | A-2 | A-5 |
| 335 | 2-2 | A-4 | A-6 | 375 | 2-2 | A-8 | A-8 | 415 | 2-3 | A-2 | A-6 |
| 336 | 2-2 | A-4 | A-7 | 376 | 2-2 | A-8 | A-9 | 416 | 2-3 | A-2 | A-7 |
| 337 | 2-2 | A-4 | A-8 | 377 | 2-2 | A-8 | A-10 | 417 | 2-3 | A-2 | A-8 |
| 338 | 2-2 | A-4 | A-9 | 378 | 2-2 | A-8 | A-11 | 418 | 2-3 | A-2 | A-9 |
| 339 | 2-2 | A-4 | A-10 | 379 | 2-2 | A-8 | A-12 | 419 | 2-3 | A-2 | A-10 |
| 340 | 2-2 | A-4 | A-11 | 380 | 2-2 | A-8 | A-13 | 420 | 2-3 | A-2 | A-11 |
| 341 | 2-2 | A-4 | A-12 | 381 | 2-2 | A-8 | A-14 | 421 | 2-3 | A-2 | A-12 |
| 342 | 2-2 | A-4 | A-13 | 382 | 2-2 | A-8 | A-15 | 422 | 2-3 | A-2 | A-13 |
| 343 | 2-2 | A-4 | A-14 | 383 | 2-2 | A-9 | A-9 | 423 | 2-3 | A-2 | A-14 |
| 344 | 2-2 | A-4 | A-15 | 384 | 2-2 | A-9 | A-10 | 424 | 2-3 | A-2 | A-15 |
| 345 | 2-2 | A-5 | A-5 | 385 | 2-2 | A-9 | A-11 | 425 | 2-3 | A-3 | A-3 |
| 346 | 2-2 | A-5 | A-6 | 386 | 2-2 | A-9 | A-12 | 426 | 2-3 | A-3 | A-4 |
| 347 | 2-2 | A-5 | A-7 | 387 | 2-2 | A-9 | A-13 | 427 | 2-3 | A-3 | A-5 |
| 348 | 2-2 | A-5 | A-8 | 388 | 2-2 | A-9 | A-14 | 428 | 2-3 | A-3 | A-6 |
| 349 | 2-2 | A-5 | A-9 | 389 | 2-2 | A-9 | A-15 | 429 | 2-3 | A-3 | A-7 |
| 350 | 2-2 | A-5 | A-10 | 390 | 2-2 | A-10 | A-10 | 430 | 2-3 | A-3 | A-8 |
| 351 | 2-2 | A-5 | A-11 | 391 | 2-2 | A-10 | A-11 | 431 | 2-3 | A-3 | A-9 |
| 352 | 2-2 | A-5 | A-12 | 392 | 2-2 | A-10 | A-12 | 432 | 2-3 | A-3 | A-10 |
| 353 | 2-2 | A-5 | A-13 | 393 | 2-2 | A-10 | A-13 | 433 | 2-3 | A-3 | A-11 |
| 354 | 2-2 | A-5 | A-14 | 394 | 2-2 | A-10 | A-14 | 434 | 2-3 | A-3 | A-12 |
| 355 | 2-2 | A-5 | A-15 | 395 | 2-2 | A-10 | A-15 | 435 | 2-3 | A-3 | A-13 |
| 356 | 2-2 | A-6 | A-6 | 396 | 2-2 | A-11 | A-11 | 436 | 2-3 | A-3 | A-14 |
| 357 | 2-2 | A-6 | A-7 | 397 | 2-2 | A-11 | A-12 | 437 | 2-3 | A-3 | A-15 |
| 358 | 2-2 | A-6 | A-8 | 398 | 2-2 | A-11 | A-13 | 438 | 2-3 | A-4 | A-4 |
| 359 | 2-2 | A-6 | A-9 | 399 | 2-2 | A-11 | A-14 | 439 | 2-3 | A-4 | A-5 |
| 360 | 2-2 | A-6 | A-10 | 400 | 2-2 | A-11 | A-15 | 440 | 2-3 | A-4 | A-6 |

**(Table 5)**

| Compound Nos. | Core | Substituent Ar1 | Substituent Ar2 | Compound Nos. | Core | Substituent Ar1 | Substituent Ar2 |
|---|---|---|---|---|---|---|---|
| 441 | 2-3 | A-4 | A-7 | 481 | 2-3 | A-8 | A-9 |
| 442 | 2-3 | A-4 | A-8 | 482 | 2-3 | A-8 | A-10 |
| 443 | 2-3 | A-4 | A-9 | 483 | 2-3 | A-8 | A-11 |
| 444 | 2-3 | A-4 | A-10 | 484 | 2-3 | A-8 | A-12 |
| 445 | 2-3 | A-4 | A-11 | 485 | 2-3 | A-8 | A-13 |
| 446 | 2-3 | A-4 | A-12 | 486 | 2-3 | A-8 | A-14 |
| 447 | 2-3 | A-4 | A-13 | 487 | 2-3 | A-8 | A-15 |
| 448 | 2-3 | A-4 | A-14 | 488 | 2-3 | A-9 | A-9 |
| 449 | 2-3 | A-4 | A-15 | 489 | 2-3 | A-9 | A-10 |
| 450 | 2-3 | A-5 | A-5 | 490 | 2-3 | A-9 | A-11 |
| 451 | 2-3 | A-5 | A-6 | 491 | 2-3 | A-9 | A-12 |
| 452 | 2-3 | A-5 | A-7 | 492 | 2-3 | A-9 | A-13 |
| 453 | 2-3 | A-5 | A-8 | 493 | 2-3 | A-9 | A-14 |
| 454 | 2-3 | A-5 | A-9 | 494 | 2-3 | A-9 | A-15 |
| 455 | 2-3 | A-5 | A-10 | 495 | 2-3 | A-10 | A-10 |
| 456 | 2-3 | A-5 | A-11 | 496 | 2-3 | A-10 | A-11 |
| 457 | 2-3 | A-5 | A-12 | 497 | 2-3 | A-10 | A-12 |
| 458 | 2-3 | A-5 | A-13 | 498 | 2-3 | A-10 | A-13 |
| 459 | 2-3 | A-5 | A-14 | 499 | 2-3 | A-10 | A-14 |
| 460 | 2-3 | A-5 | A-15 | 500 | 2-3 | A-10 | A-15 |
| 461 | 2-3 | A-6 | A-6 | 501 | 2-3 | A-11 | A-11 |
| 462 | 2-3 | A-6 | A-7 | 502 | 2-3 | A-11 | A-12 |
| 463 | 2-3 | A-6 | A-8 | 503 | 2-3 | A-11 | A-13 |
| 464 | 2-3 | A-6 | A-9 | 504 | 2-3 | A-11 | A-14 |
| 465 | 2-3 | A-6 | A-10 | 505 | 2-3 | A-11 | A-15 |
| 466 | 2-3 | A-6 | A-11 | 506 | 2-3 | A-12 | A-12 |
| 467 | 2-3 | A-6 | A-12 | 507 | 2-3 | A-12 | A-13 |
| 468 | 2-3 | A-6 | A-13 | 508 | 2-3 | A-12 | A-14 |
| 469 | 2-3 | A-6 | A-14 | 509 | 2-3 | A-12 | A-15 |
| 470 | 2-3 | A-6 | A-15 | 510 | 2-3 | A-13 | A-13 |
| 471 | 2-3 | A-7 | A-7 | 511 | 2-3 | A-13 | A-14 |
| 472 | 2-3 | A-7 | A-8 | 512 | 2-3 | A-13 | A-15 |
| 473 | 2-3 | A-7 | A-9 | 513 | 2-3 | A-14 | A-14 |
| 474 | 2-3 | A-7 | A-10 | 514 | 2-3 | A-14 | A-15 |
| 475 | 2-3 | A-7 | A-11 | 515 | 2-3 | A-15 | A-15 |
| 476 | 2-3 | A-7 | A-12 | | | | |
| 477 | 2-3 | A-7 | A-13 | | | | |
| 478 | 2-3 | A-7 | A-14 | | | | |
| 479 | 2-3 | A-7 | A-15 | | | | |
| 480 | 2-3 | A-8 | A-8 | | | | |

The compound represented by Chemical Formula 2 may be, for example represented by Chemical Formula 2-A to 2-C.

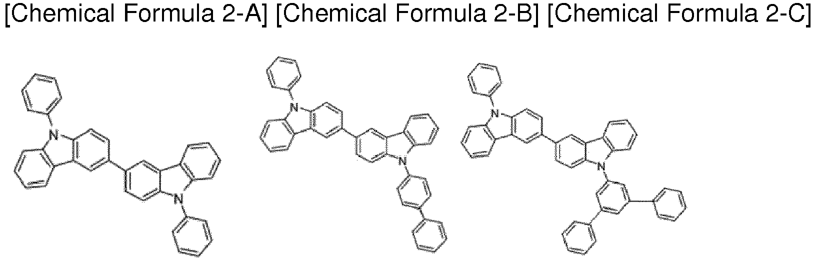

The second host compound may be, for example a compound represented by Chemical Formula 3. 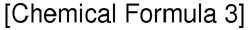 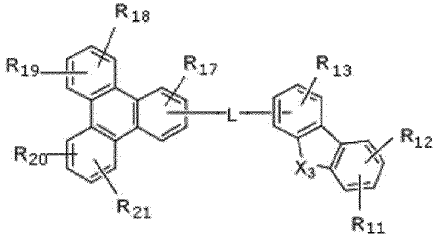
In Chemical Formula 3,
X₃ is O, S, CRₑR_{f}, SiR_{g}Rₕ, or NRᵢ,
L is a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, or a combination thereof, and
R₁₁ to R₁₃, R₁₇ to R₂₁, and Rₑ to Rᵢ are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.

The compound represented by Chemical Formula 3 may be, for example represented by Chemical Formula 3a according to a bonding position.

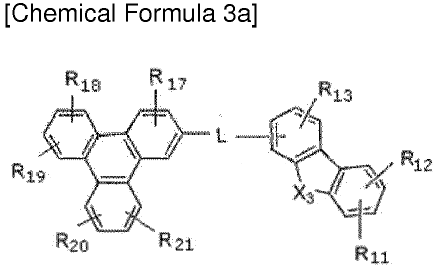

In Chemical Formula 3a, X₃, L, R₁₁ to R₁₃, R₁₇ to R₂₁, and Rₑ to Rᵢ are the same as described above.

The compound represented by Chemical Formula 3 may be, for example represented by one of Chemical Formulae 3-1 to 3-10.

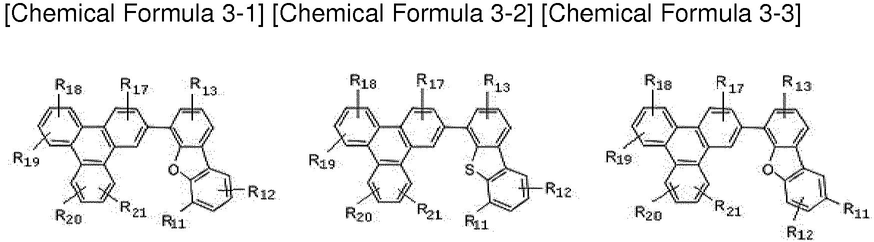

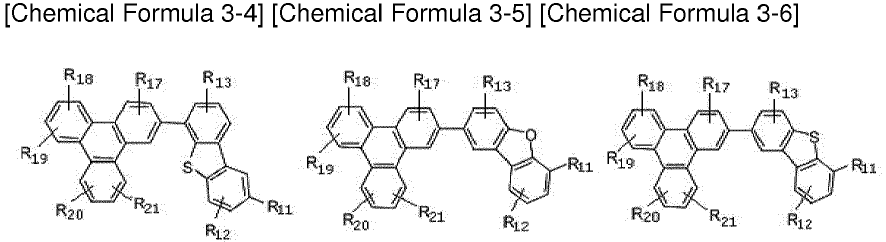

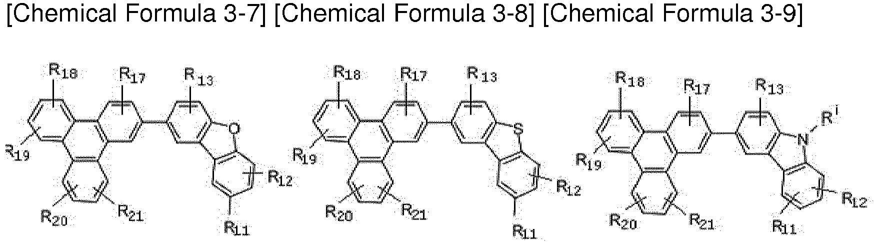

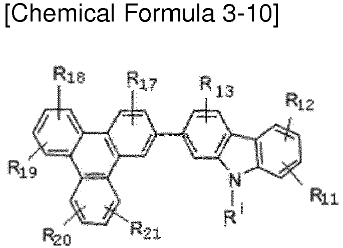

In Chemical Formulae 3-1 to 3-10, R₁₁ to R₁₃, R₁₇ to R₂₁, and Rᵢ are the same as described above.

At least one among R₁₁ to R₁₃, R₁₇ to R₂₁, and Rₑ to Rᵢ in Chemical Formulae 3, 3a, and 3-1 to 3-10 may be a substituent having one hole, for example, a substituent selected from Chemical Formulae A-1 to A-15.

The compound represented by Chemical Formula 3 may be, for example, represented in [Table 6] and [Table 7], wherein the substituent indicates R₁₁ when a core moiety is 3-1 to 3-8 and the substituent indicates Rⁱ when the core moiety is 3-9 and 3-10, and particularly, other non-mentioned substituents are all hydrogen.

**(Table 6)**

| Compound Nos. | Core moiety | Substituent | Compound Nos. | Core moiety | Substituent | Compound Nos. | Core moie ty | Substituent |
|---|---|---|---|---|---|---|---|---|
| 516 | 3-1 | A-1 | 556 | 3-3 | A-12 | 596 | 3-6 | A-8 |
| 517 | 3-1 | A-2 | 557 | 3-3 | A-13 | 597 | 3-6 | A-9 |
| 518 | 3-1 | A-3 | 558 | 3-3 | A-14 | 598 | 3-6 | A-10 |
| 519 | 3-1 | A-4 | 559 | 3-3 | A-15 | 599 | 3-6 | A-11 |
| 520 | 3-1 | A-5 | 560 | 3-4 | A-2 | 600 | 3-6 | A-12 |
| 521 | 3-1 | A-6 | 561 | 3-4 | A-3 | 601 | 3-6 | A-13 |
| 522 | 3-1 | A-7 | 562 | 3-4 | A-4 | 602 | 3-6 | A-14 |
| 523 | 3-1 | A-8 | 563 | 3-4 | A-5 | 603 | 3-6 | A-15 |
| 524 | 3-1 | A-9 | 564 | 3-4 | A-6 | 604 | 3-7 | A-2 |
| 525 | 3-1 | A-10 | 565 | 3-4 | A-7 | 605 | 3-7 | A-3 |
| 526 | 3-1 | A-11 | 566 | 3-4 | A-8 | 606 | 3-7 | A-4 |
| 527 | 3-1 | A-12 | 567 | 3-4 | A-9 | 607 | 3-7 | A-5 |
| 528 | 3-1 | A-13 | 568 | 3-4 | A-10 | 608 | 3-7 | A-6 |
| 529 | 3-1 | A-14 | 569 | 3-4 | A-11 | 609 | 3-7 | A-7 |
| 530 | 3-1 | A-15 | 570 | 3-4 | A-12 | 610 | 3-7 | A-8 |
| 531 | 3-2 | A-1 | 571 | 3-4 | A-13 | 611 | 3-7 | A-9 |
| 532 | 3-2 | A-2 | 572 | 3-4 | A-14 | 612 | 3-7 | A-10 |
| 533 | 3-2 | A-3 | 573 | 3-4 | A-15 | 613 | 3-7 | A-11 |
| 534 | 3-2 | A-4 | 574 | 3-5 | A-1 | 614 | 3-7 | A-12 |
| 535 | 3-2 | A-5 | 575 | 3-5 | A-2 | 615 | 3-7 | A-13 |
| 536 | 3-2 | A-6 | 576 | 3-5 | A-3 | 616 | 3-7 | A-14 |
| 537 | 3-2 | A-7 | 577 | 3-5 | A-4 | 617 | 3-7 | A-15 |
| 538 | 3-2 | A-8 | 578 | 3-5 | A-5 | 618 | 3-8 | A-2 |
| 539 | 3-2 | A-9 | 579 | 3-5 | A-6 | 619 | 3-8 | A-3 |
| 540 | 3-2 | A-10 | 580 | 3-5 | A-7 | 620 | 3-8 | A-4 |
| 541 | 3-2 | A-11 | 581 | 3-5 | A-8 | 621 | 3-8 | A-5 |
| 542 | 3-2 | A-12 | 582 | 3-5 | A-9 | 622 | 3-8 | A-6 |
| 543 | 3-2 | A-13 | 583 | 3-5 | A-10 | 623 | 3-8 | A-7 |
| 544 | 3-2 | A-14 | 584 | 3-5 | A-11 | 624 | 3-8 | A-8 |
| 545 | 3-2 | A-15 | 585 | 3-5 | A-12 | 625 | 3-8 | A-9 |
| 546 | 3-3 | A-2 | 586 | 3-5 | A-13 | 626 | 3-8 | A-10 |
| 547 | 3-3 | A-3 | 587 | 3-5 | A-14 | 627 | 3-8 | A-11 |
| 548 | 3-3 | A-4 | 588 | 3-5 | A-15 | 628 | 3-8 | A-12 |
| 549 | 3-3 | A-5 | 589 | 3-6 | A-1 | 629 | 3-8 | A-13 |
| 550 | 3-3 | A-6 | 590 | 3-6 | A-2 | 630 | 3-8 | A-14 |
| 551 | 3-3 | A-7 | 591 | 3-6 | A-3 | 631 | 3-8 | A-15 |
| 552 | 3-3 | A-8 | 592 | 3-6 | A-4 | 632 | 3-9 | A-2 |
| 553 | 3-3 | A-9 | 593 | 3-6 | A-5 | 633 | 3-9 | A-3 |
| 554 | 3-3 | A-10 | 594 | 3-6 | A-6 | 634 | 3-9 | A-4 |
| 555 | 3-3 | A-11 | 595 | 3-6 | A-7 | 635 | 3-9 | A-5 |

**(Table 7)**

| Compound Nos. | Core moiety | Substituent | Compound Nos. | Core moiety | Substituent | Compound Nos. | Core moiety | substituent |
|---|---|---|---|---|---|---|---|---|
| 636 | 3-9 | A-6 | 644 | 3-9 | A-14 | 652 | 3-10 | A-8 |
| 637 | 3-9 | A-7 | 645 | 3-9 | A-15 | 653 | 3-10 | A-9 |
| 638 | 3-9 | A-8 | 646 | 3-10 | A-2 | 654 | 3-10 | A-10 |
| 639 | 3-9 | A-9 | 647 | 3-10 | A-3 | 655 | 3-10 | A-11 |
| 640 | 3-9 | A-10 | 648 | 3-10 | A-4 | 656 | 3-10 | A-12 |
| 641 | 3-9 | A-11 | 649 | 3-10 | A-5 | 657 | 3-10 | A-13 |
| 642 | 3-9 | A-12 | 650 | 3-10 | A-6 | 658 | 3-10 | A-14 |
| 643 | 3-9 | A-13 | 651 | 3-10 | A-7 | 659 | 3-10 | A-15 |

The compound represented by Chemical Formula 3 may be for example represented by one of Chemical Formulae 3-A to 3-E.

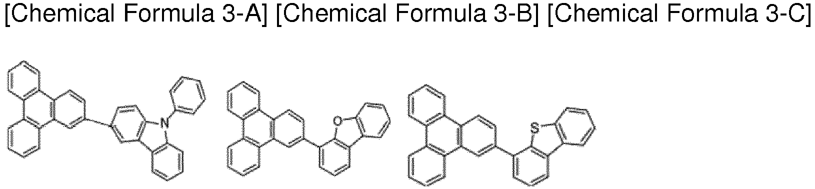

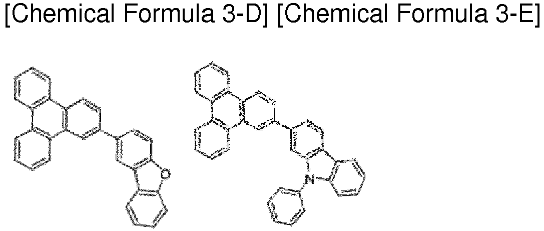

The first and second host compounds may be variously mixed to prepare various compositions. For example, the composition may include the first host compound and the second host compound represented by Chemical Formula 2, and another composition may include the first host compound and the third host compound represented by Chemical Formula 3.

For example, the composition may include a compound selected from compounds shown in Chemical Formula I in Tables 1 and 2 as a first host compound and a compound represented by Chemical Formula 2 in Tables 3 to 5 or a compound represented by Chemical Formula 3 in Tables 6 and 7 as a second host compound.

The compounds listed in Tables 1 to 7 show various first host and second host compounds, but are not limited thereto.

As described above, since the first host compound simultaneously has electron characteristics, and the second host compound has hole characteristics, these two compouns may be included together to realize bipolar characteristics. Accordingly, when the composition is used to form an emission layer for an organic optoelectronic device, satisfactory interface characteristics and hole and electron-transporting capability may be improved.

The first and second host compounds may be, for example, included in a weight ratio of about 1:10 to about 10:1. When the first and second host compounds are included within the range, bipolar characteristics and improve efficiency and a life-span may be realized by suing electron transport capability of the first host compound and hole transport capability of the second host compound in an appropriate ratio to form an emission layer.

The composition may further include at least one host compound in addition to the first host compound and the second host compound.

The composition may further include a dopant. The dopant may be a red, green, or blue dopant, for example, a phosphorescent dopant.

The dopant is mixed with the first host compound and the second host compound in a small amount to cause light emission, and may be generally a material such as a metal complex that emits light by multiple excitation into a triplet or more. The dopant may be, for example an inorganic, organic, or organic/inorganic compound, and one or more kinds thereof may be used.

Examples of the phosphorescent dopant may be an organic metal compound including Ir, Pt, Os, Ti, Zr, Hf, Eu, Tb, Tm, Fe, Co, Ni, Ru, Rh, Pd or a combination thereof. The phosphorescent dopant may be, for example compounds represented by Chemical Formulae A to C, but is not limited thereto.

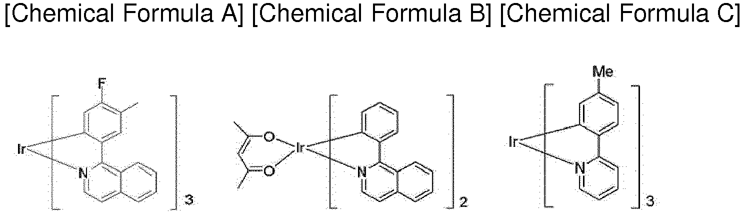

The composition may form a film using a dry film-forming method such as chemical vapor deposition.

Hereinafter, an organic optoelectronic device to which the composition is applied is described.

The organic optoelectronic device may be any device to convert electrical energy into photoenergy and vice versa without particular limitation, and may be, for example an organic photoelectric device, an organic light emitting diode, an organic solar cell, and an organic photo-conductor drum.

Herein, an organic light emitting diode as one example of an organic optoelectronic device is described referring to drawings.

FIGS. 1 and 2 are cross-sectional views of each organic light emitting diode according to one embodiment.

Referring to FIG. 1, an organic optoelectronic device 100 according to one embodiment includes an anode 120 and a cathode 110 facing each other and an organic layer 105 interposed between the anode 120 and cathode 110.

The anode 120 may be made of a conductor having a high work function to help hole injection, and may be for example metal, metal oxide and/or a conductive polymer. The anode 120 may be, for example a metal nickel, platinum, vanadium, chromium, copper, zinc, gold, and the like or an alloy thereof; metal oxide such as zinc oxide, indium oxide, indium tin oxide (ITO), indium zinc oxide (IZO), and the like; a combination of metal and oxide such as ZnO and Al or SnO2 and Sb; a conductive polymer such as poly(3-methylthiophene), poly(3,4-(ethylene-1,2-dioxy)thiophene) (PEDT), polypyrrole, and polyaniline, but is not limited thereto.

The cathode 110 may be made of a conductor having a low work function to help electron injection, and may be for example metal, metal oxide and/or a conductive polymer. The cathode 110 may be for example a metal or an alloy thereof such as magnesium, calcium, sodium, potassium, titanium, indium, yttrium, lithium, gadolinium, aluminum silver, tin, lead, cesium, barium, and the like; a multi-layer structure material such as LiF/Al, LiO₂/Al, LiF/Ca, LiF/Al and BaF₂/Ca, but is not limited thereto.

The organic layer 105 includes an emission layer 130 including the above-mentioned composition.

The emission layer 130 may include for example the above-mentioned composition.

Referring to FIG. 2, an organic light emitting diode 200 further includes a hole auxiliary layer 140 as well as an emission layer 130. The hole auxiliary layer 140 may further increase hole injection and/or hole mobility between the anode 120 and emission layer 130 and block electrons. The hole auxiliary layer 140 may be, for example a hole transport layer, a hole injection layer, and/or an electron blocking layer, and may include at least one layer.

In one embodiment of the present invention, an organic light emitting diode may further include an electron transport layer, an electron injection layer (EIL), a hole injection layer, and the like, as an organic thin layer 105 in FIG. 1 or FIG. 2.

The organic light emitting diodes 100 and 200 may be manufactured by forming an anode or a cathode on a substrate, forming an organic layer in accordance with a dry coating method such as evaporation, sputtering, plasma plating, and ion plating; and forming a cathode or an anode thereon.

The organic light emitting diode may be applied to an organic light emitting diode (OLED) display.

### [Mode for Invention]

Hereinafter, the embodiments are illustrated in more detail with reference to examples. However, these examples are exemplary, and the present disclosure is not limited thereto.

### Synthesis of Organic Compound

### Synthesis of Intermediate 1-1

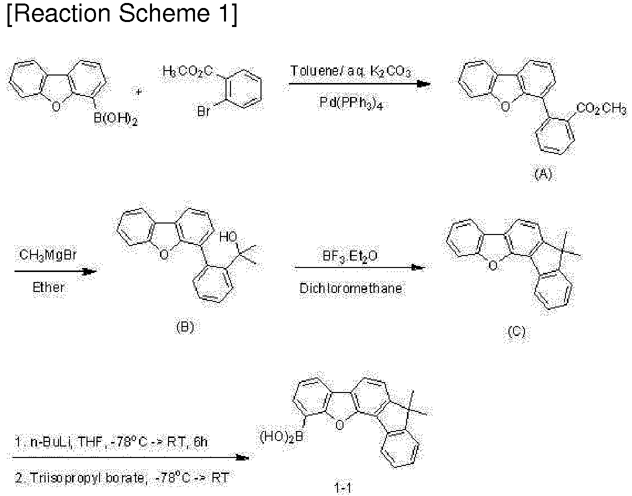

### First step; Synthesis of Intermediate Product (A)

23g (108.5 mmol) of an intermediate, (4-dibenzofuranyl)boronic acid, 24.5 g (113.9 mmol) of methyl-2-bromo-benzoate, and 6.3 g (5.47 mmol) of tetrakistriphenylphosphine palladium were put in a flask and dissolved in 500 ml of toluene under a nitrogen atmosphere, 271.2 ml of an aqueous solution in which 79.9 g (542.4 mmol) of potassium carbonate was dissolved was added thereto, and the mixture was refluxed and agitated for 12 hours. When the reaction was complete, the resultant was extracted with ethylacetate, dried with magnesium sulfate, filtered, and concentrated under a reduced pressure. Then, a product therefrom was purified with n-hexane/dichloromethane (7:3 of a volume ratio) through silica gel column chromatography, obtaining 31 g (a yield of 94.5 %) of a desired compound, an intermediate A as a white solid.

### LC-Mass (Theoretical value: 302.32 g/mol, Measured value: M+1 = 302 g/mol)

### Second Step; Synthesis of Intermediate Product (B)

29 g (95.92 mmol) of the intermediate A was put in a flask and dissolved in 400 ml of anhydrous tetrahydrofuran (THF) under a nitrogen atmosphere, and the solution was cooled down to 0 °C and agitated. Then, 95.9 mL (287.8 mmol) of 3M methyl magnesium bromide (in diethyl ether) was slowly added thereto, and the mixture was agitated at room temperature under a nitrogen atmosphere for 5 hours. When the reaction was complete, the solution was concentrated under a reduced pressure to remove a solvent therein, and an extract obtained by using distilled water and dichloromethane was dried with magnesium sulfate, filtered, and concentrated under a reduced pressure, obtaining a desired compound, an intermediate (B) as a liquid having high viscosity.

### Third Step; Synthesis of Intermediate Product (C)

The intermediate (B) was dissolved in 250 ml of dichloromethane, and the solution was cooled down to 0 °C and agitated. Then, borontrifluoride dissolved in 20 mL of dichloromethane and 12.84 g (47.5 mmol) of diethyl ether complex were slowly added thereto, and the mixture was agitated at room temperature for 5 hours. When the reaction was complete, the resultant was extracted with ethylacetate, an extract was dried with magnesium sulfate, filtered, and concentrated under a reduced pressure. Then, a product therefrom was purified with n-Hexane through silica gel column chromatography, obtaining 22 g (yield of 71.9 %) of a desired compound, an intermediate (C) as a white solid.

### LC-Mass (Theoretical value: 284.35 g/mol, Measured value: M+1 = 284 g/mol)

### Fourth Step; Synthesis of Intermediate Product 1-1

70 g (246.18 mmol) of the intermediate (C) was put in a thermally-dried two-necked round-bottomed flask under vacuum, 500 mL of anhydrous tetrahydrofuran was added thereto under a nitrogen atmosphere to dissolve it, and the solution was cooled down to - 78 °C and agitated. Herein, 118 mL (295.41 mmol) of 2.5 M n-butyllithium (in hexane) was slowly added thereto, and the mixture was agitated at room temperature under a nitrogen atmosphere for 6 hours. The reaction solution was cooled down to -78 °C, 68.17 mL (295.41 mmol) of triisopropyl borate was slowly added thereto, and the mixture was agitated at room temperature for 12 hours. The reaction was completed with a 3N HCl aqueous solution, the resultant was extracted with ethylacetate, and an extracted was dried with magnesium sulfate, dried, filtered, and concentrated under a reduced pressure. Then, a product therefrom was purified with a silica gel filter, obtaining 62.5 g (a yield of 77 %) of a desired compound, an intermediate 1-1.

### LC-Mass (Theoretical value: 328.17 g/mol, Measured value: M+1 = 328 g/mol)

### Synthesis of Intermediate 1-2

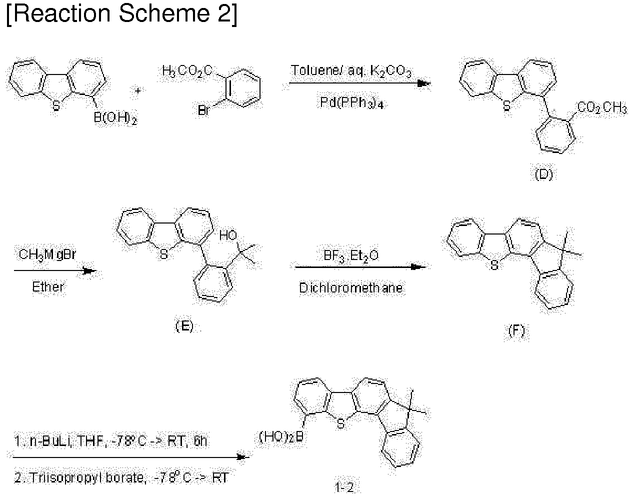

### First Step; Synthesis of Intermediate Product (D)

25 g (109.62 mmol) of an intermediate, (4-dibenzothiophenyl)boronic acid, 24.8 g (115.1 mmol) of methyl-2-bromo-benzoate, and 6.3 g (5.48 mmol) of tetrakistriphenylphosphine palladium were put in a flask and dissolved in 500 ml of toluene under a nitrogen atmosphere, 274 ml of an aqueous solution in which 80.7 g (548.1 mmol) of potassium carbonate was dissolved was added thereto, and the mixture was refluxed and agitated for 12 hours. When the reaction was complete, the resultant was extracted with ethylacetate, and an extract was dreid with magnesium sulfate, filtered, and concentrated under a reduced pressure. Then, a product therefrom was purified with n-hexane/dichloromethane (7:3 of a volume ratio) through silica gel column chromatography, obtaining 31 g (a yield of 88.8 %) of a desired compound, an intermediate (D) as a white solid.

### GC-Mass (Theoretical value: 318.39 g/mol, Measured value: M+1 = 318 g/mol)

### Second Step; Synthesis of Intermediate Product (E)

15.4 g (47.1 mmol) of the intermediate (D) was put in a flask and dissolved in 400 ml of anhydrous tetrahydrofuran under a nitrogen atmosphere, and the solution was cooled down to 0 °C and agitated. Then, 50 mL (141.34 mmol) of 3 M methyl magnesium bromide (in diethyl ether) was slowly added thereto, and the mixture was agitated at room temperature under a nitrogen atmosphere for 5 hours. When the reaction was complete, the resultant was concentrated under a reduced pressure to remove a solvent therein and extracted with distilled water and dichloromethane, an extract was dried with magnesium sulfate and filtered, and the filtered solution was concentrated under a reduced pressure, obtaining a desired compound, an intermediate (E) as a liquid having high viscosity.

### Third Step; Synthesis of Intermediate Product (F)

The intermediate (E) was dissolved in 250 ml of dichloromethane, and the solution was cooled down to 0 °C and agitated. Then, borontrifluoride dissolved in 20 mL of dichloromethane and 12.6 g (47.1 mmol) of a diethyl ether complex were slowly added thereto, and the mixture was agitated at room temperature for 5 hours. When the reaction was complete, the resultant was extracted with ethylacetate, an extract was dried with magnesium sulfate and filtered, and a filtered solution was concentrated under a reduced pressure. Then, a product therefrom was purified with n-hexane through silica gel column chromatography, obtaining 10.5 g (a yield of 74.3 %) of a desired compound, an intermediate (F) as a white solid.

### LC-Mass (Theoretical value: 300.42 g/mol, Measured value: M+1 = 300 g/mol)

### Fourth Step; Synthesis of Intermediate Product 1-2

9.6 g (31.79 mmol) of the intermediate (F) was put under a nitrogen atmosphere in a two-necked round-bottomed flask thermally dried under vacuum and dissolved in 100 mL of anhydrous tetrahydrofuran, and the solution was cooled down to -78 °C and agitated. Then, 20.7 mL (63.6 mmol) of 1.6 M n-butyllithium (in hexane) was slowly added thereto, and the mixture was agitated at room temperature under a nitrogen atmosphere for 6 hours. The reaction solution was cooled down to -78 °C, 8.8 mL (38.15 mmol) of triisopropyl borate was slowly added thereto, and the mixture was agitated at room temperature for 12 hours. The reaction was completed with a 3N HCl aqueous solution, the resultant was extracted with ethylacetate, and an extract was dried with magnesium sulfate, filtered, and concentracted under a reduced pressure. Then, a product therefrom was purified with a silica gel filter, obtaining 8.47 g (a yield of 77 %) of a desired compound, an intermediate 1-2.

### LC-Mass (Theoretical value: 344.23 g/mol, Measured value: M+1 = 344 g/mol)

### Synthesis of Intermediate 1-3

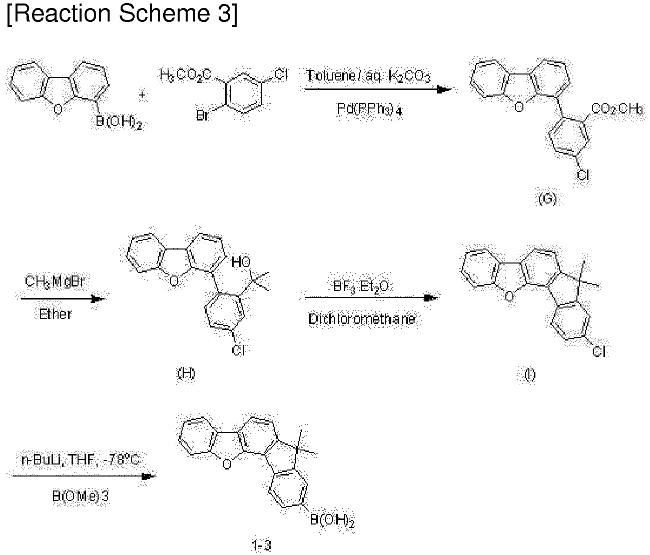

### First Step; Synthesis of Intermediate Product (G)

30 g (141.5 mmol) of (4-dibenzofuranyl)boronic acid, 37.1 g (148.6 mmol) of methyl-2-bromo-5-chlorobenzoate, and 8.2 g (7.1 mmol) of tetrakistriphenylphosphine palladium were put in a flask and then, dissolved in 550 ml of toluene under a nitrogen atmosphere, 353.8 ml of an aqueous solution in which 104.2 g (707.51 mmol) of potassium carbonate was dissolved was added thereto, and the mixture was refluxed and agitated for 12 hours. When the reaction was complete, the resultant was extracted with ethylacetate, and an extract was dried with magnesium sulfate, filtered, and concentrated under reduced pressure. Then, a product therefrom was purified with n-hexane/dichloromethane (7: 3 of a volume ratio) through silica gel column chromatography, obtaining 38.2 g (a yield of 80 %) of a desired compound, an intermediate (G) as a white solid.

### LC-Mass (Theoretical value: 336.06 g/mol, Measured value: M+1 = 336 g/mol)

### Second Step; Synthesis of Intermediate Product (H)

38.18 g (113.37 mmol) of the intermediate (G) was put in a flask and dissolved in 500 ml of anhydrous ether under a nitrogen atmosphere, and the solution was cooled down to 0 °C and agitated. Then, 110 mL (340.1 mmol) of 3 M methyl magnesium bromide (in diethyl ether) was slowly added thereto, and the mixture was agitated at room temperature under a nitrogen atmosphere for 5 hours. When the reaction was complete, the solution was concentrated under a reduced pressure to remove a solvent therefrom and then, extracted with distilled water and dichloromethane, and an extract obtained therefrom was dried with anhydrous magnesium sulfate, filtered, and concentrated under a reduced pressure, obtaining a desired compound, an intermediate (H) as a liquid having high viscosity.

### Third Step; Synthesis of Intermediate Product (I)

The intermediate (H) was dissolved in 250 ml of dichloromethane, and the solution was cooled down to 0 °C and agitated. Then, borontrifluoride dissolved in 20 mL of dichloromethane and 15.18 g (56.7 mmol) of a diethyl ether complex were slowly added thereto, and the mixture was agitated at room temperature for 5 hours. When the reaction was complete, the resultant was extracted with ethylacetate, an extract was dried with magnesium sulfate, filtered, and concentrated under a reduced pressure. Then, a product therefrom was purified with n-hexane through silica gel column chromatography, obtaining 29 g (a yield of 80 %) of a desired compound, an intermediate (I) as a white solid.

### GC-Mass (Theoretical value: 318.8 g/mol, Measured value: M+1 = 318 g/mol)

### Fourth Step; Synthesis of Intermediate Product 1-3

29 g (90.97 mmol) of the intermediate (I) was put under a nitrogen atmosphere in a two-necked round-bottomed flask thermally dried under vacuum and dissolved in 300 mL of anhydrous tetrahydrofuran, and the solution was cooled down to -78 °C and agitated. Then, 68.2 mL (109.16 mmol) of 1.6 M n-butyllithium (in hexane) was slowly thereto, and the mixture was agitated at room temperature under a nitrogen atmosphere for 6 hours. The reaction solution was cooled down to -78 °C, 7.61 mL (109.16 mmol) of trimethyl borate was slowly added thereto, and the mixture was agitated at room temperature for 12 hours. The reaction was completed by a 3N HCl aqueous solution and extracted with ethylacetate, and an extract was dried with magnesium sulfate, filtered, and concentrated under a reduced pressure. Then, a product therefrom was purified with a silica gel filter, obtaining 23 g (a yield of 77 %) of a desired compound, an intermediate 1-3.

### Synthesis of Intermediate 1-4

An intermediate 1-4 was synthesized by using (4-dibenzothienyl)boronic acid as a synthesis starting material through the same steps in Reaction Scheme 4 as the intermediate 1-3.

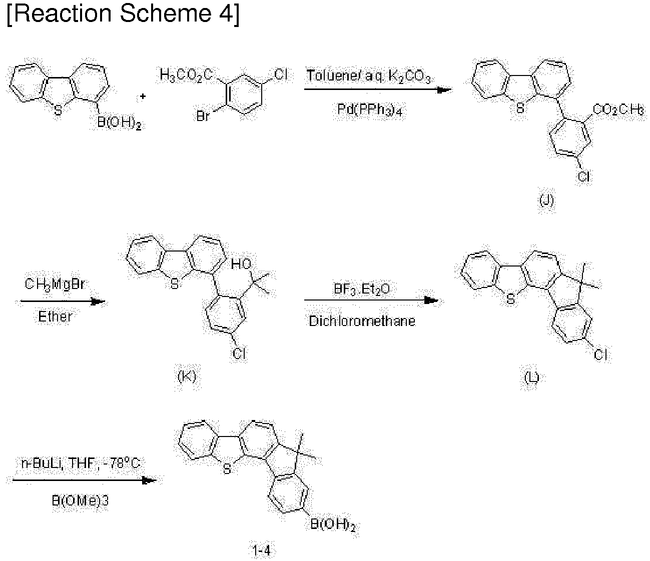

### Synthesis of Intermediate 1-5

An intermediate 1-5 was synthesized by using methyl-2-bromo-4-chlorobenzoate as a synthesis starting material through the same steps in Reaction Scheme 4 as the intermediate 1-3.

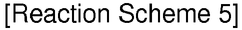

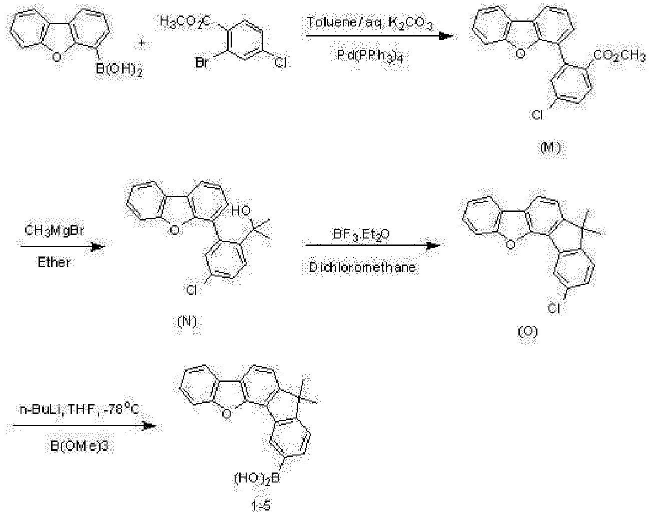

### Synthesis of Intermediate 1-6

An intermediate 1-6 was synthesized by using (4-dibenzothienyl)boronic acid as a synthesis starting material through the same steps in Reaction Scheme 6 as the intermediate 1-5.

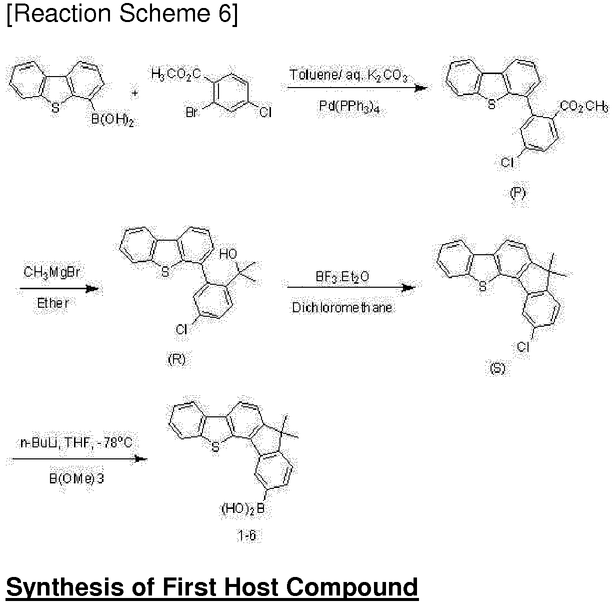

### Example 1: Synthesis of First Host Chemical Formula [1-F]

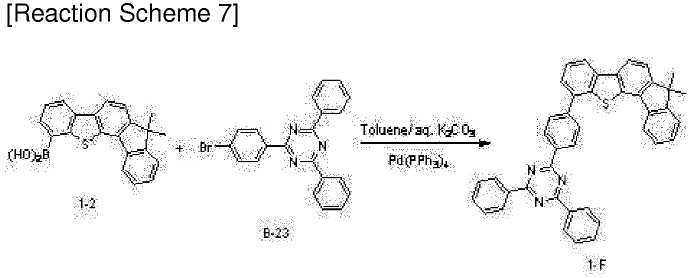

10 g (29.1 mmol) of the intermediate 1-2, 10.3 g (26.4 mmol) of the compound B-23, 10.95 g (79.23 mmol) of potassium carbonate, and 0.61 g (0.53 mmmol) of tetrakis-(triphenylphosphine)palladium (0) were suspended in 100 ml of toluene and 40 ml of distilled water and then, refluxed and agitated for 12 hours. The resultant was extracted with dichloromethane and distilled water, and an organic layer was silica gel-filtered. Then, an organic solution was removed therefrom, its residue was silica gel columned with hexane:dichloromethane = 7 : 3 (v/v), and a solid product was recrystallized with dichloromethane and n-hexane, obtaining 14 g (a yield : 87 %) of a compound represented by Chemical Formula 1-F.

### Examples 2 to 16: Preparation of Compounds

Compounds respectively represented by Chemical Formula 1-A to 1-E and 1-G to 1-P were synthesized in the same method as Example 1 except for using the compounds arranged in Tables 8-1 to 8-3 instead of the intermediate 1-2 and the compound B-23 in Example 1.

**[Table 8-1]**

| **Ex** | **Starting material 1** | **Starting material 2** | **Product** | Yield(%) LC-Mass (M+H⁻). |
|---|---|---|---|---|
| 2 | 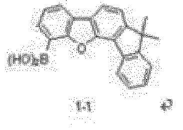 | 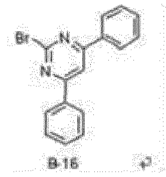 | 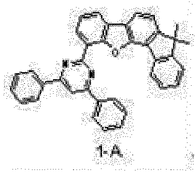 | 82 % |
| | | | | 515.21 |
| 3 | 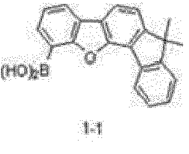 | 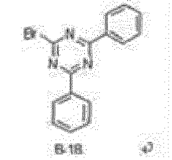 | 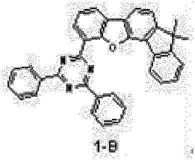 | 80 % |
| | | | | 516.20 |
| 4 | 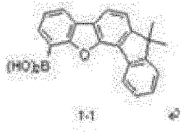 | 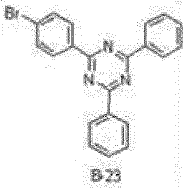 | 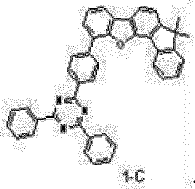 | 78 % |
| | | | | 592.23 |
| 5 | 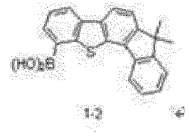 | 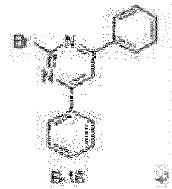 | 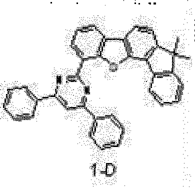 | 85 % |
| | | | | 531.19 |
| 6 | 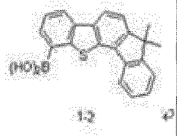 | 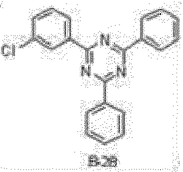 | 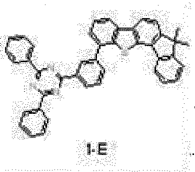 | 79 % |
| | | | | 608.21 |
| 7 | 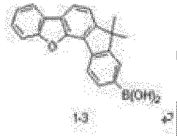 | 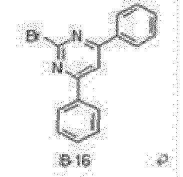 | 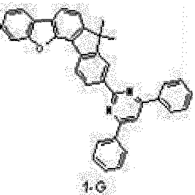 | 88 % |
| | | | | 515.21 |

**[Table 8-2]**

| | | | | |
|---|---|---|---|---|
| 8 | 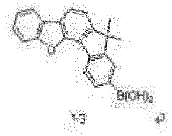 | 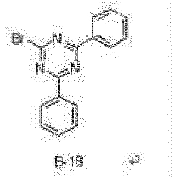 | 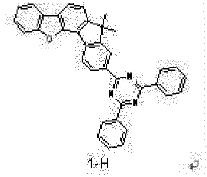 | 89 % |
| | | | | 516.20 |
| 9 | 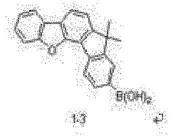 | 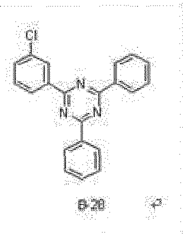 | 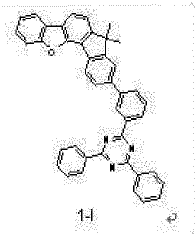 | 82 % |
| | | | | 592. |
| 10 | 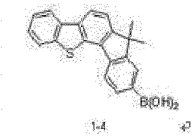 | 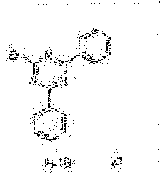 | 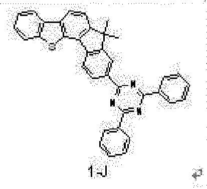 | 18 % |
| | | | | 532.18 |
| 11 | 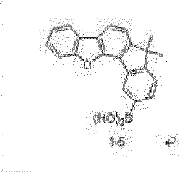 | 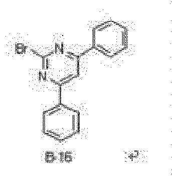 | 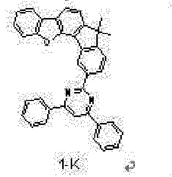 | 87 % |
| | | | | 515.21 |
| 12 | 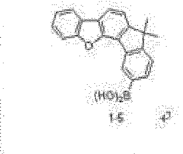 | 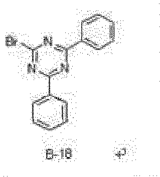 | 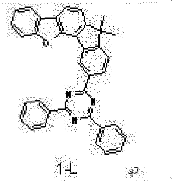 | 85 % |
| | | | | 516.20 |
| 13 | 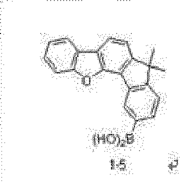 | 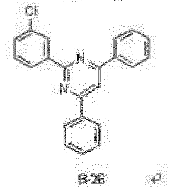 | 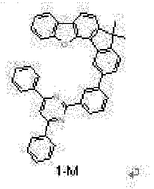 | 77 % |
| | | | | 591.24 |

**[Table 8-3]**

| | | | | |
|---|---|---|---|---|
| 14 | 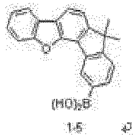 | 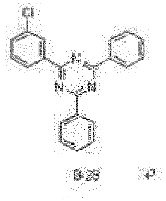 | 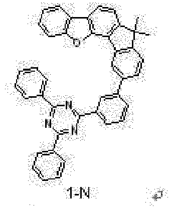 | 80 % |
| | | | | 592.23 |
| 15 | 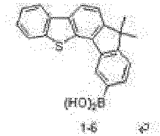 | 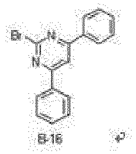 | 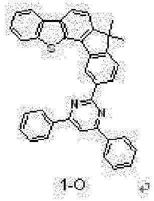 | 84 % |
| | | | | 531.19 |
| 16 | 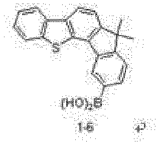 | 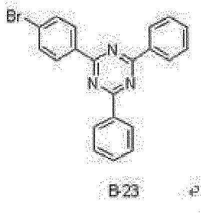 | 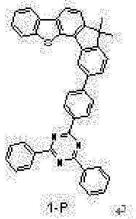 | 80 % |
| | | | | 608.21 |

### Synthesis of Second Host Compound

### Example 17: Synthesis of Second Host Chemical Formula [2-A]

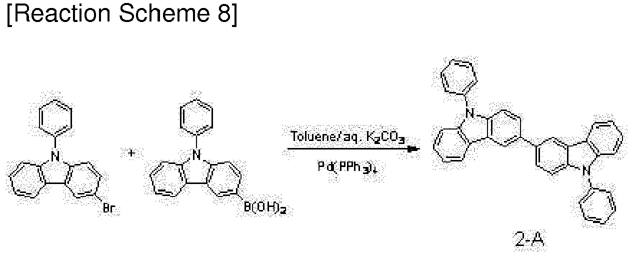

9.97 g (30.95 mmol) of penylcarbazolylbromide, 9.78 g (34.05 mmol) of phenylcarbazolylboronic acid, 12.83 g (92.86 mmol) of potassium carbonate, and 1.07 g (0.93 mmmol) of tetrakis-(triphenylphosphine)palladium (0) were suspended in 120 ml of toluene and 50 ml of distilled water and then, refluxed and agiated for 12 hours. The resultant was extracted with dichloromethane and distilled water, and an organic layer was silica gel-filtered. Then, an organic solution was removed therefrom, and a solid product therefrom was recrystallized with dichloromethane and n-hexane, obtaining 13.8 g (a yield: 92 %) of a compound represented by Chemical Formula 2-A.

### Examples 18 and 19: Preparation of Compounds

A compound was prepared according to the same method as above except for using two starting materials provided in the following Table instead of Phenylcarbazolylbromide (corresponding to a starting material 1 in Table 9) and phenylcarbazolylboronic acid (corresponding to a starting material 2 in Table 9) of Example 17.

**[Table 9]**

| **Ex** | **Starting material 1** | **Starting material 2** | **Product** | **Yield (%)** LC-Mass (M+H⁺) |
|---|---|---|---|---|
| 18 | 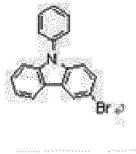 | 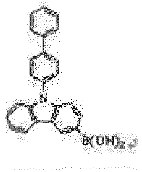 | 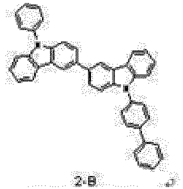 | 90 % |
| | | | | 561.23 |
| 19 | 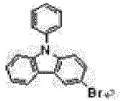 | 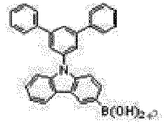 | 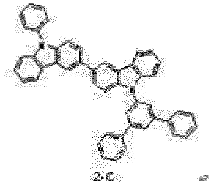 | 85 % |
| | | | | 637.27 |

### Example 20: Synthesis of Second Host Chemical Formula [3-A]

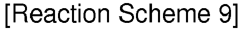

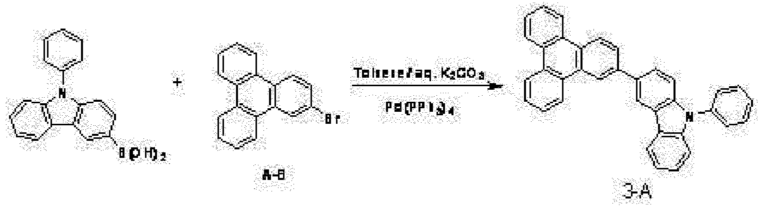

10 g (34.83 mmol) of phenylcarbazolylboronic acid, 11.77 g (38.31 mmol) of the compound A-8, 14.44 g (104.49 mmol) of potassium carbonate, and 0.80 g (0.7 mmmol) of tetrakis-(triphenylphosphine)palladium (0) were suspended in 140 ml of toluene and 50 ml of distilled water and then, refluxed and agitated for 12 hours. The resultant was extracted with dichloromethane and distilled water, and an organic layer was silica gel-filtered. After removing an organic solution therefrom, its residue was silica gel columned with hexane : dichloromethane = 7 : 3 (v/v), and a solid product therefrom was recrystallized with dichloromethane and n-hexane, obtaining 14.4 g (a yield : 88 %) of a compound represented by Chemical Formula 3-A.

### Examples 21 to 24: Preparation of Compounds

Each compound respectively represented by Chemical Formulae 3-B to 3-E was synthesized according to the same method as Example 20 except for using compounds arranged in Table 10 instead of the phenylcarbazoleyl boronic acid and the compound A-8 of Example 20.

**[Table 10]**

| **Ex** | **Starting material 1** | **Starting material 2** | **Product** | Yield(%) LC-Mass M+H⁻) |
|---|---|---|---|---|
| 21 | 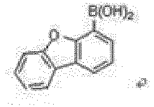 | 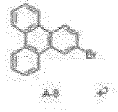 | 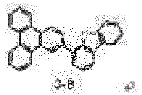 | 89 % |
| | | | | 395.14 |
| 22 | 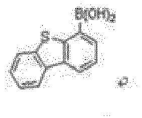 | 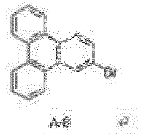 | 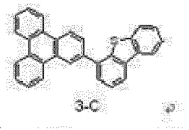 | 90 % |
| | | | | 411.12 |
| 23 | 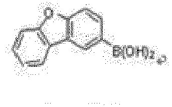 | 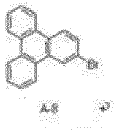 | 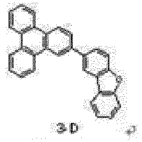 | 92 % |
| | | | | 395.14 |
| 24 | 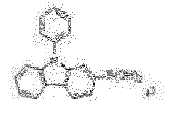 | 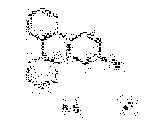 | 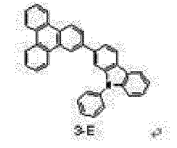 | 90 % |
| | | | | 470.19 |

### Manufacture of Organic Light Emitting Diode

### Example 25

As for an anode, 1000 Å-thick ITO was used, and as for a cathode, 1000 Å-thick aluminum (Al) was used. Specifically, illustrating a method of manufacturing the organic light emitting diode, the anode is manufactured by cutting an ITO glass substrate having 15 Ω/cm² of sheet resistance into a size of 50 mm × 50 mm × 0.7 mm, ultrasonic wave-cleaning them in acetone, isopropylalcohol, and pure water for 15 minutes respectively, and UV ozone cleaning them for 30 minutes.

On the substrate, an 800 Å-thick hole transport layer (HTL) was formed by depositing N4,N4'-di(naphthalen-1-yl)-N4,N4'-diphenylbiphenyl-4,4'-diamine (NPB) under a vacuum degree 650×10⁻⁷ Pa at a deposit speed of 0.1 to 0.3 nm/s. Subsequently, a 300 Å-thick emission layer was formed through a vacuum depositon by mixing the compound 1-F of Example 1 and the compound 2-A of Example 17 which are obtained under the same vacuum deposit conditio in a weight ratio of 1:1 as a host and simultaneously, doping the mixture with a phosphorescent dopant of Ir(ppy)3. Herein, the phosphorescent dopant was deposited in an amount of 10 wt% based on 100 wt% of the total amount of the emission layer by adjusting its deposit speed.

On the emission layer, a 50 Å-thick hole blocking layer was formed by depositing bis(2-methyl-8-quinolinolate)-4-(phenylphenolato)aluminium (BAlq) under the same vacuum deposit condition. Subsequently, a 250 Å-thick electron transport layer was formed by depositing Alq3 under the same vacuum deposit condition. On the electron transport layer, a cathode was formed by sequentially depositing LiF and Al, manufacturing an organic photoelectric device.

The organic photoelectric device had a structure of ITO/ NPB (80 nm)/ EML (compound 1-F (45 wt%) + compound 2-A (45 wt%) + Ir(PPy)3 (10 wt%), 30 nm)/ Balq (5 nm)/ Alq3 (25 nm)/ LiF (1 nm) / Al (100 nm).

### Example 26

An organic light emitting diode was manufactured according to the same method as Example 25 except for using the compound 1-F and the compound 2-A in a ratio of 4:1 instead of mixing the compound 1-F and the compound 2-A in a weight ratio of 1:1.

### Example 27

An organic light emitting diode was manufactured according to the same method as Example 25 except for using the compound 1-F and the compound 2-A in a ratio of 1:4 instead of mixing the compound 1-F and the compound 2-A in a weight ratio of 1:1.

### Example 28

An organic light emitting diode was manufactured according to the same method as Example 25 except for depositing the compound 1-N according to Example 14 and the compound 2-A in a ratio of 1:1 instead of mixing the compound 1-F and the compound 2-A in a weight ratio of 1:1.

### Example 29

An organic light emitting diode was manufactured according to the same method as Example 28 except for depositing the compound 1-N and the compound 2-A in a ratio of 4:1 instead of mixing the compound 1-N and the compound 2-A in a weight ratio of 1:1.

### Example 30

An organic light emitting diode was manufactured according to the same method as Example 28 except for depositing the compound 1-N and the compound 2-A in a ratio of 1:4 instead of mixing the compound 1-N and the compound 2-A in a weight ratio of 1:1.

### Example 31

An organic light emitting diode was manufactured according to the same method as Example 28 except for depositing the compound 1-N and the compound 3-A according to Example 22 in a ratio of 1:1 instead of mixing the compound 1-N and the compound 2-A in a weight ratio of 1:1.

### Example 32

An organic light emitting diode was manufactured according to the same method as Example 31 except for depositing the compound 1-N and the compound 3-A in a ratio of 4:1 instead of mixing the compound 1-N and the compound 3-A in a weight ratio of 1:1.

### Example 33

An organic light emitting diode was manufactured according to the same method as Example 31 except for depositing the compound 1-N and the compound 3-A in a ratio of 1:4 instead of mixing the compound 1-N and the compound 3-A in a weight ratio of 1:1.

### Comparative Example 1

An organic light emitting diode was manufactured according to the same method as Example 25 except for depositing the compound 1-F as a single host instead of mixing the compound 1-F and the compound 2-A in a weight ratio of 1:1.

### Comparative Example 2

An organic light emitting diode was manufactured according to the same method as Example 28 except for depositing the compound 1-N as a single host instead of mixing the compound 1-N and the compound 2-A in a weight ratio of 1:1.

### Comparative Example 3

An organic light emitting diode was manufactured according to the same method as Example 28 except for depositing the compound 2-A as a single host instead of mixing the compound 1-N and the compound 2-A in a weight ratio of 1:1.

### Comparative Example 4

An organic light emitting diode was manufactured according to the same method as Example 31 except for depositing the compound 3-A as a host instead of mixing the compound 1-N and the compound 2-A in a weight ratio of 1:1.

### Evaluation

Current density, luminance changes and luminous efficiency depending on a voltage of each organic light emitting diode according to Examples 25 to 33 and Comparative Examples 1 to 4 were measured.

The measurements were specifically performed in the following method, and the results were provided in Table.

### (1) Measurement of Current Density Change Depending on Voltage Change

Current values flowing in the unit device of the manufactured organic light emitting diodes were measured for, while increasing the voltage from 0V to 10V using a current-voltage meter (Keithley 2400), and the measured current values were divided by an area to provide the results.

### (2) Measurement of Luminance Change Depending on Voltage Change

Luminance of the manufactured organic light emitting diodes was measured for luminance, while increasing the voltage from 0 V to 10 V using a luminance meter (Minolta Cs-1000A).

### (3) Measurement of Luminous Efficiency

Current efficiency (cd/A) at the same current density (10 mA/cm2) were calculated by using the luminance, current density, and voltages (V) from the items (1) and (2).

### (4) Measurement of Life-span

Life-span was obtained by measuring time taken until current efficiency (cd/A) decreased down to 95 % while luminance (cd/m²) was maintained at 5000 cd/m².

**(Table 11)**

| | Compound of emission layer | Driving voltage (V) | Color (EL color) | Efficie ncy (cd/A) | 95 % life-span (h) @ 5000 cd/m² |
|---|---|---|---|---|---|
| Example 25 | 1-F + 2-A (1:1) | 3.96 | Green | 53.3 | 300 |
| Example 26 | 1-F + 2-A (4:1) | 3.81 | Green | 38.2 | 220 |
| Example 27 | 1-F + 2-A (1:4) | 5.02 | Green | 39.4 | 80 |
| Example 28 | 1-N + 2-A (1:1) | 4.26 | Green | 53.4 | 240 |
| Example 29 | 1-N + 2-A (4:1) | 4.24 | Green | 36.9 | 260 |
| Example 30 | 1-N + 2-A (1:4) | 8.44 | Green | 25.0 | - |
| Example 31 | 1-N + 3-A (1:1) | 4.77 | Green | 38.8 | 380 |
| Example 32 | 1-N + 3-A (4:1) | 4.56 | Green | 27.8 | 180 |
| Example 33 | 1-N + 3-A (1:4) | 5.66 | Green | 47.3 | 200 |
| Comparative Example 1 | 1-F | 4.29 | Green | 23.6 | 170 |
| Comparative Example 2 | 1-N | 4.82 | Green | 21.0 | 90 |
| Comparative Example 3 | 2-A | 7.13 | Green | 1.7 | - |
| Comparative Example 4 | 3-A | 8.68 | Green | 16.5 | 20 |

Referring to Table 11, the organic light emitting diodes according to Examples 25 to 33 showed remarkably improved luminous efficiency or/ 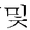 life-span compared with the organic light emitting diodes according to Comparative Examples 1 to 4. Specifically, the organic light emitting diode according to Example 31 showed improved efficiency and superbly improved life-span compared with the organic light emitting diodes according to Comparative Examples 2 and 4. The reason is that the efficiency and the life-span were improved by bipolar characteristics of a hole-transporting host and an electron-transporting host.

## Claims

1. A composition comprising a first host compound represented by Chemical Formula I and a second host compound represented by Chemical Formula II: 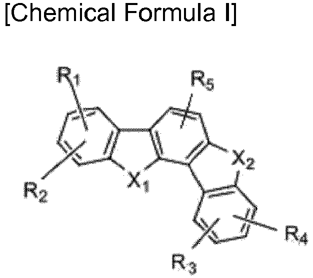
wherein, in Chemical Formula I,
X₁ is O, Se, S, SO, SO₂, PO, or CO,
X₂ is CRₐR_{b} or SiR_{c}R_{d}, and
R₁ to R₅ and Rₐ to R_{d} are each independently hydrogen, deuterium, a substituted or unsubstituted C1 to C30 alkyl group, a substituted or unsubstituted C3 to C30 cycloalkyl group, a substituted or unsubstituted C3 to C30 heterocycloalkyl group, a substituted or unsubstituted C6 to C30 aryl group, a substituted or unsubstituted C3 to C30 heteroaryl group, a substituted or unsubstituted amine group, a substituted or unsubstituted C6 to C30 arylamine group, a substituted or unsubstituted C3 to C30 heteroarylamine group, a substituted or unsubstituted C1 to C30 alkoxy group, a substituted or unsubstituted C2 to C30 alkoxycarbonyl group, a substituted or unsubstituted C2 to C30 alkoxycarbonylamino group, a substituted or unsubstituted C7 to C30 aryloxycarbonylamino group, a substituted or unsubstituted C1 to C30 sulfamoylamino group, a substituted or unsubstituted C2 to C30 alkenyl group, a substituted or unsubstituted C2 to C30 alkynyl group, a substituted or unsubstituted silyl group, a substituted or unsubstituted silyloxy group, a substituted or unsubstituted C1 to C30 acyl group, a substituted or unsubstituted C1 to C20 acyloxy group, a substituted or unsubstituted C1 to C20 acylamino group, a substituted or unsubstituted C1 to C30 sulfonyl group, a substituted or unsubstituted C1 to C30 alkylthiol group, a substituted or unsubstituted C1 to C30 heterocyclothiol group, a substituted or unsubstituted C6 to C30 arylthiol group, a substituted or unsubstituted C1 to C30 heteroarylthiol group, a substituted or unsubstituted C1 to C30 ureide group, a halogen, a halogen-containing group, a cyano group, a hydroxyl group, an amino group, a nitro group, a carboxyl group, a ferrocenyl group, or a combination thereof, 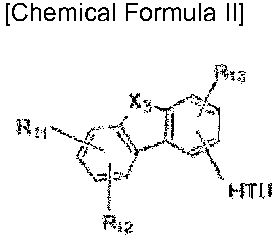
wherein in Chemical Formula II,
X₃ is O, S, CRᵤRᵥ, SiR_{w}Rₓ, or NR_{y},
HTU is a substituted or unsubstituted carbazolyl group or a substituted or unsubstituted triphenylenyl group, and
R₁₁ to R₁₃ and Rᵤ to R_{y} are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.

2. The composition of claim 1, wherein the first host compound is represented by one of Chemical Formulae 1a to 1c: 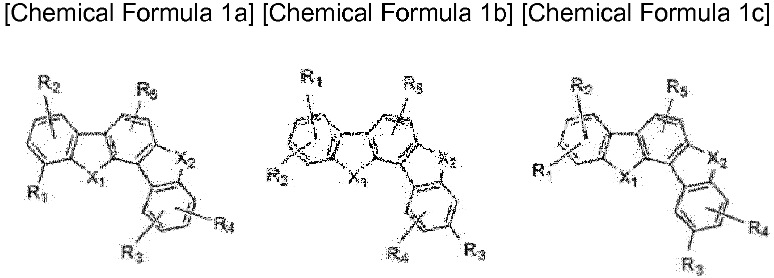
wherein, in Chemical Formulae 1a to 1c,
X₁ is O, Se, S, SO, SO₂, PO, or CO,
X₂ is CRₐR_{b} or SiR_{c}R_{d}, and
R₁ to R₅ and Rₐ to R_{d} are each independently hydrogen, deuterium, a substituted or unsubstituted C1 to C30 alkyl group, a substituted or unsubstituted C3 to C30 cycloalkyl group, a substituted or unsubstituted C3 to C30 heterocycloalkyl group, a substituted or unsubstituted C6 to C30 aryl group, a substituted or unsubstituted C3 to C30 heteroaryl group, a substituted or unsubstituted amine group, a substituted or unsubstituted C6 to C30 arylamine group, a substituted or unsubstituted C3 to C30 heteroarylamine group, a substituted or unsubstituted C1 to C30 alkoxy group, a substituted or unsubstituted C2 to C30 alkoxycarbonyl group, a substituted or unsubstituted C2 to C30 alkoxycarbonylamino group, a substituted or unsubstituted C7 to C30 aryloxycarbonylamino group, a substituted or unsubstituted C1 to C30 sulfamoylamino group, a substituted or unsubstituted C2 to C30 alkenyl group, a substituted or unsubstituted C2 to C30 alkynyl group, a substituted or unsubstituted silyl group, a substituted or unsubstituted silyloxy group, a substituted or unsubstituted C1 to C30 acyl group, a substituted or unsubstituted C1 to C20 acyloxy group, a substituted or unsubstituted C1 to C20 acylamino group, a substituted or unsubstituted C1 to C30 sulfonyl group, a substituted or unsubstituted C1 to C30 alkylthiol group, a substituted or unsubstituted C1 to C30 heterocyclothiol group, a substituted or unsubstituted C6 to C30 arylthiol group, a substituted or unsubstituted C1 to C30 heteroarylthiol group, a substituted or unsubstituted C1 to C30 ureide group, a halogen, a halogen-containing group, a cyano group, a hydroxyl group, an amino group, a nitro group, a carboxyl group, a ferrocenyl group or a combination thereof.

3. The composition of claim 1, wherein the first host compound is represented by one of Chemical Formulae 1-1 to 1-6: 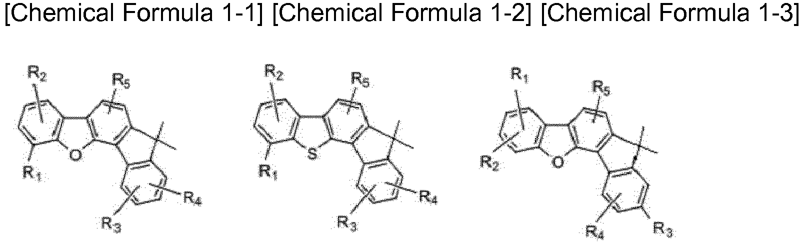 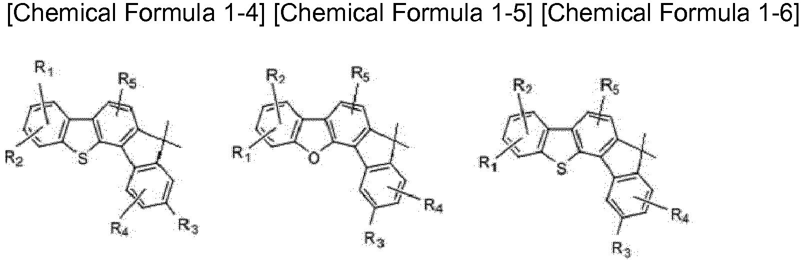
wherein, in Chemical Formulae 1-1 to 1-6,
R₁ to R₅ are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.

4. The composition of claim 1, wherein at least one of R₁ to R₅ and Rₐ to R_{d} is selected from substituted or unsubstituted functional groups of Group 1: 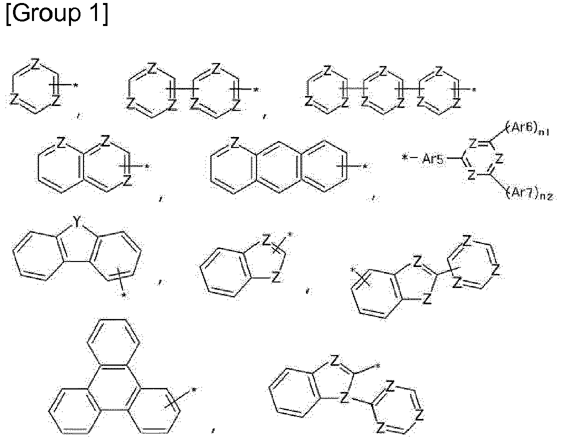
wherein, in the Group 1,
Y is N, O, S, SO₂, NRⱼ, CRₖ, SiRₗ, CRₘRₙ or SiRₒRₚ,
Z is N, CR_{q}, CRᵣRₛ or NRₜ,
wherein Rⱼ to Rₜ are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof,
Ar5 is a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, a substituted or unsubstituted C6 to C30 arylamine group, a substituted or unsubstituted C2 to C30 heteroarylamine group, or a combination thereof,
Ar6 and Ar7 are independently substituted or unsubstituted C6 to C30 aryl group, a substituted or unsubstituted C3 to C30 heteroaryl group, or a combination thereof,
n1 and n2 are each independently 0 or 1, and
* is a linking point to Chemical Formula 1 and may be positioned at one of elements of the functional group.

5. The composition of claim 4, wherein the substituted or unsubstituted functional group of Group 1 is selected from substituted or unsubstituted Chemical Formulae B-1 to B-35:

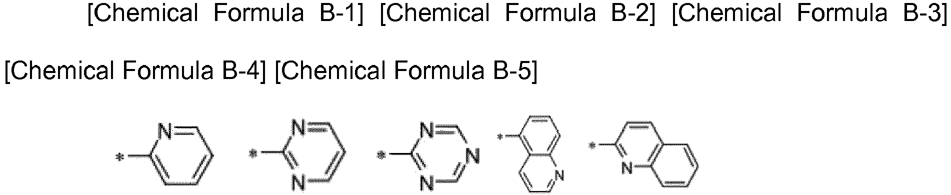

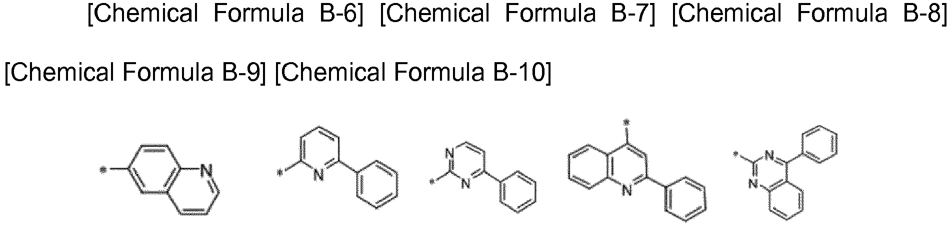

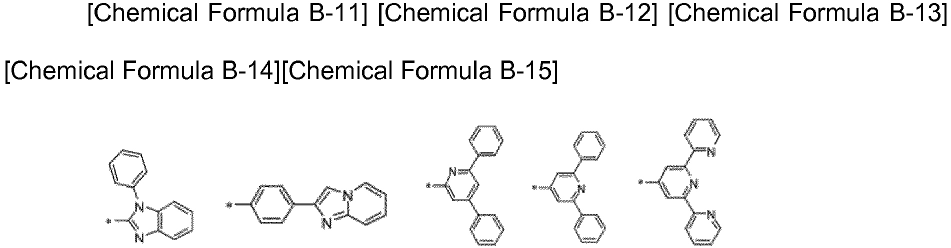

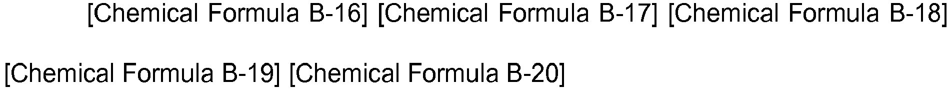

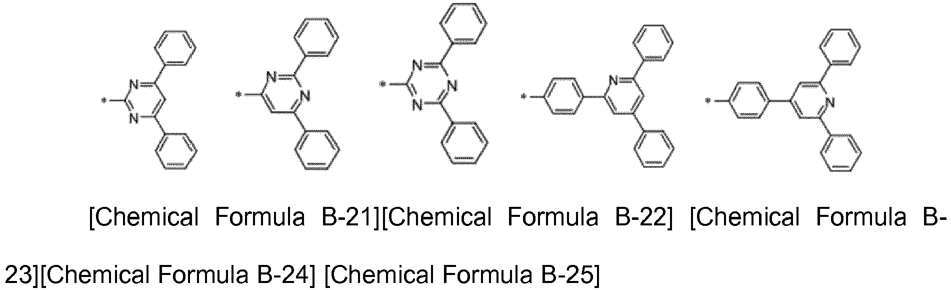

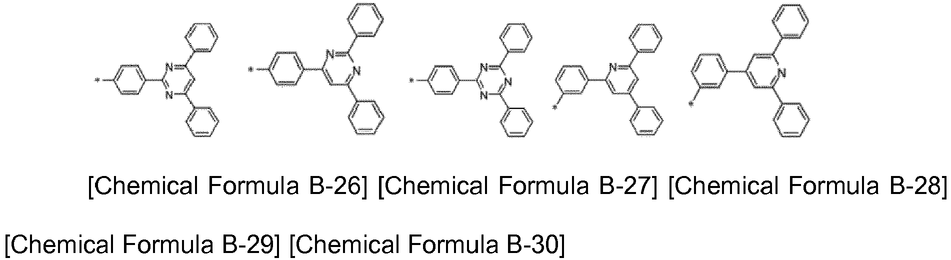

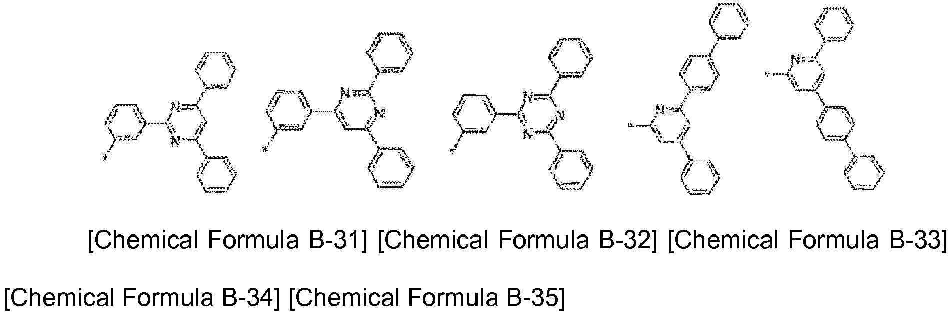

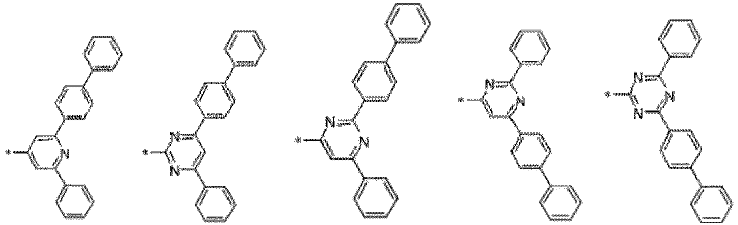

6. The composition of claim 1, wherein the first host compound is selected from Chemical Formulae 1-A to 1-P:

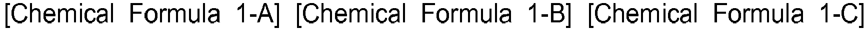

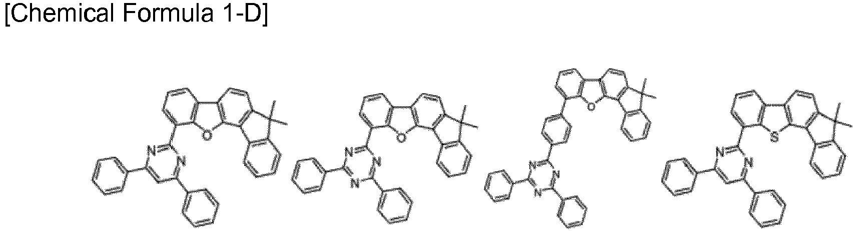

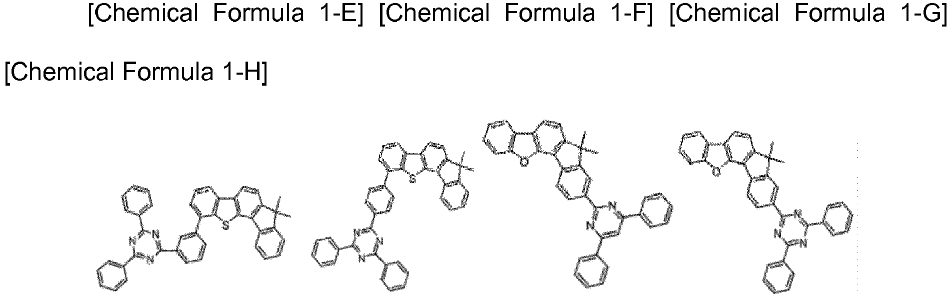

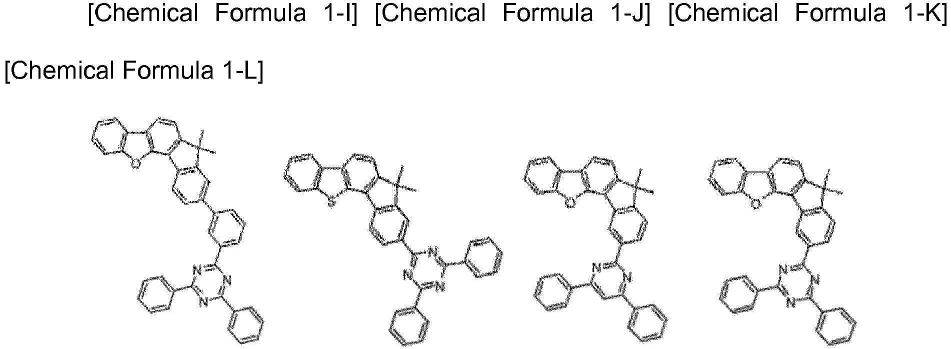

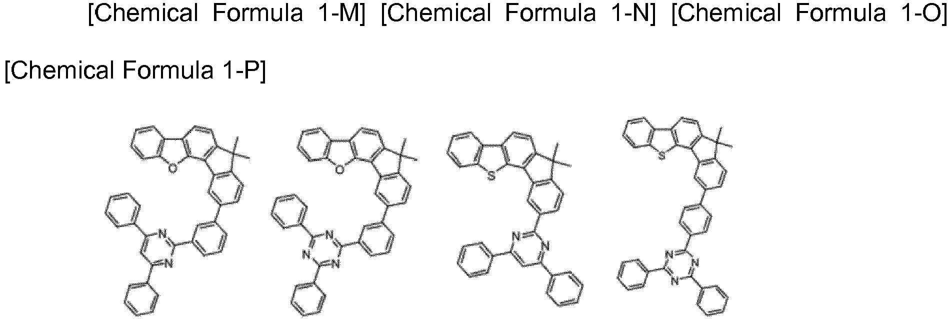

7. The composition of claim 1, wherein the second host compound is represented by Chemical Formula 2: 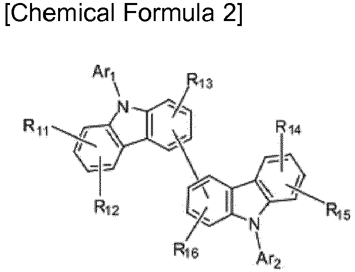
wherein, in Chemical Formula 2,
Ar₁, Ar₂ and R₁₁ to R₁₆ are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.

8. The composition of claim 7, wherein the second host compound is represetnted by one of Chemical Formulae 2-1 to 2-3: 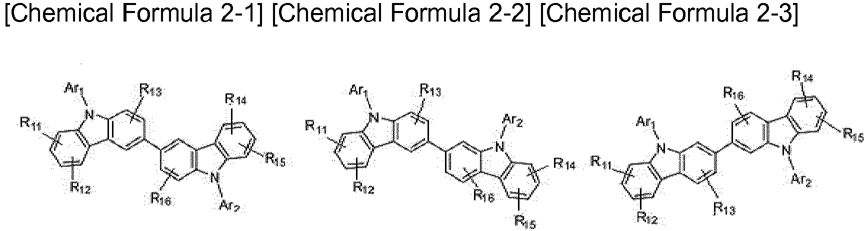
wherein, in Chemical Formulae 2-1 to 2-3,
Ar₁, Ar₂ and R₁₁ to R₁₆ are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.

9. The composition of claim 7, wherein Ar₁ and Ar₂ of Chemical Formula 2 are each independently selected from substituted or unsubstituted Chemical Formulae A-1 to A-15:

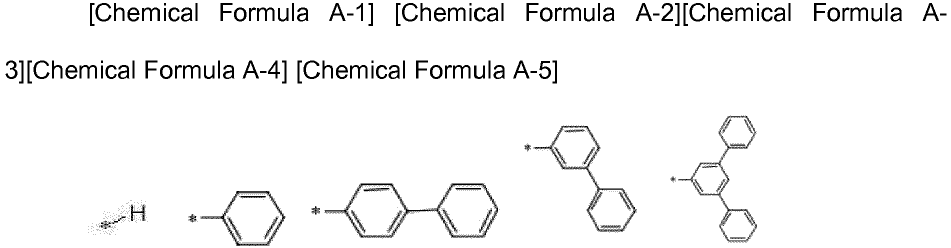

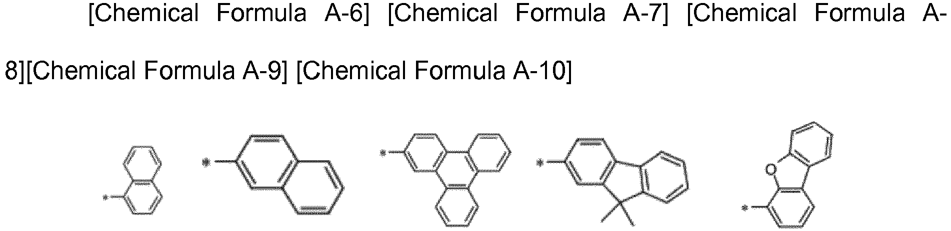

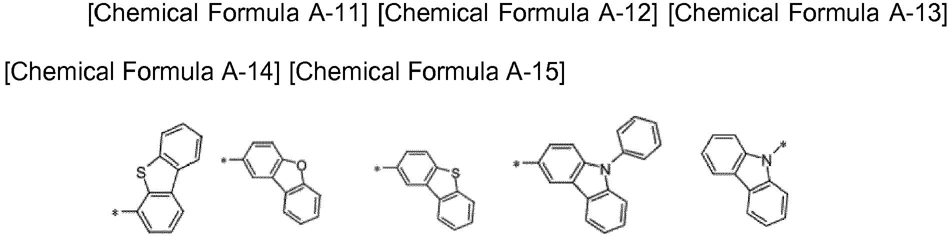

10. The composition of claim 1, wherein the second host compound is represented by Chemical Formula 3: 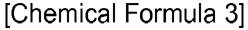 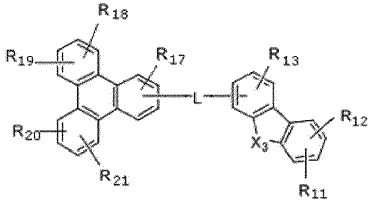
wherein, in Chemical Formula 3,
X₃ is O, S, CRₑR_{f}, SiR_{g}Rₕ or NRᵢ,
L is a single bond, a substituted or unsubstituted C1 to C20 alkylene group, a substituted or unsubstituted C2 to C20 alkenylene group, a substituted or unsubstituted C6 to C30 arylene group, a substituted or unsubstituted C2 to C30 heteroarylene group, or a combination thereof, and
R₁₁ to R₁₃, R₁₇ to R₂₁, and Rₑ to Rᵢ are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.

11. The composition of claim 10, wherein the second host compound is represented by one of Chemical Formulae 3-1 to 3-10: 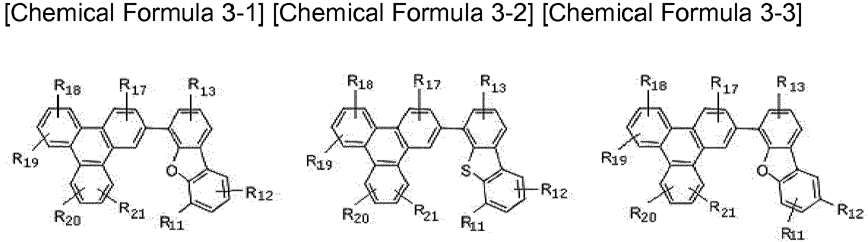 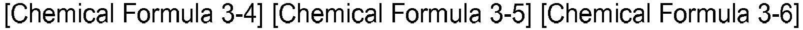 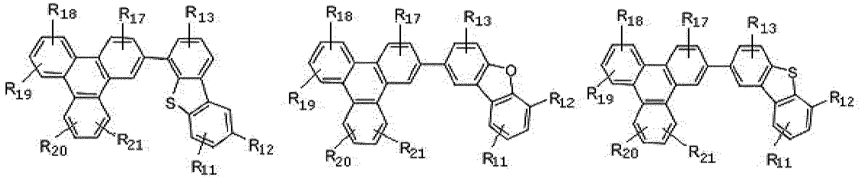 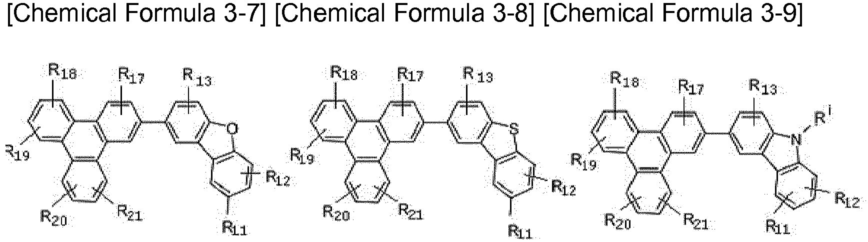 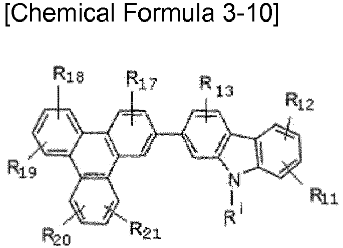
wherein in Chemical Formulae 3-1 to 3-10,
R₁₁ to R₁₃, R₁₇ to R₂₁, and Rᵢ are each independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.

12. The composition of claim 10, wherein at least one of R₁₁ to R₁₃, R₁₇ to R₂₁, and Rₑ to Rᵢ is selected from substituted or unsubstituted Chemical Formulae A-1 to A-15.

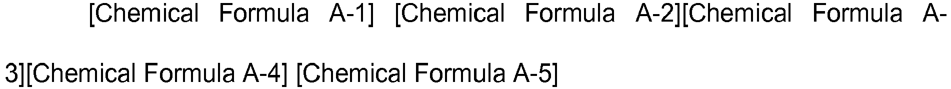

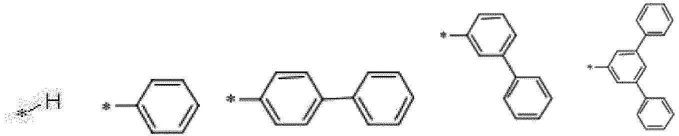

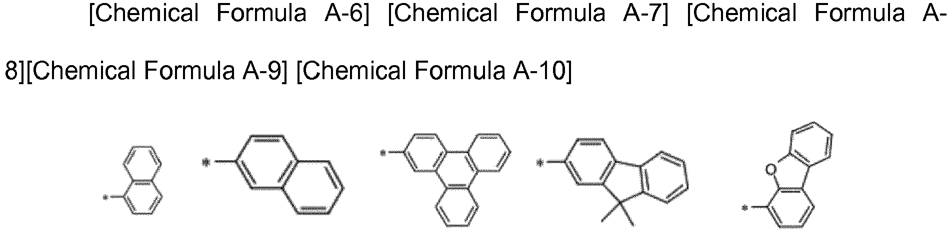

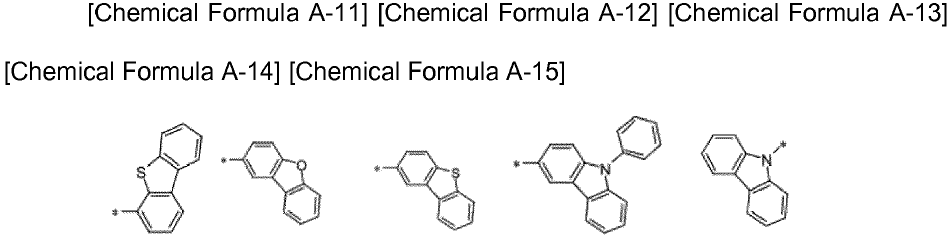

13. The composition of claim 1, wherein the second host compound is represented by Chemical Formulae 2-A to 2-C, Chemical Formulae 3-A to 3-D or 3-E:

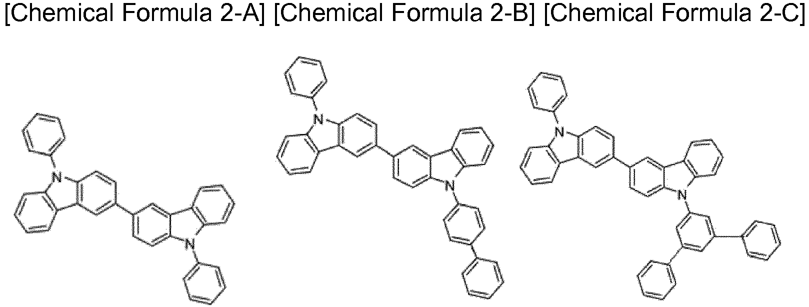

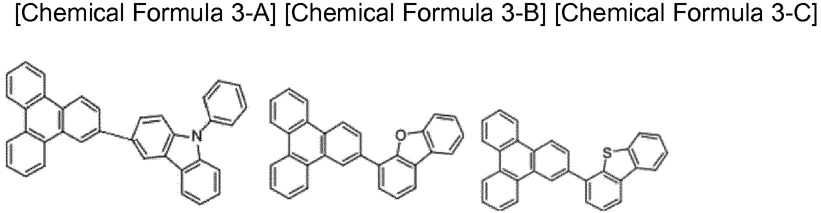

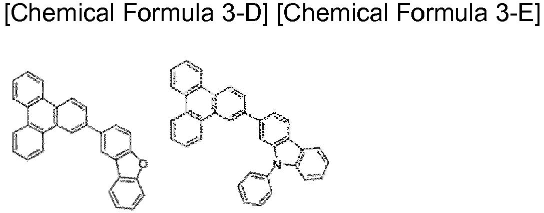

14. The composition of claim 1, wherein the first host compound and the second host compound are included in a weight ratio of 1:10 to 10:1.

15. An organic optoelectric device comprising
an anode and a cathode facing each other, and
at least one organic layer interposed between the anode and the cathode,
wherein the organic layer comprises the composition of any one of claim 1 to claim 14.

## Patentansprüche

1. Zusammensetzung, umfassend eine erste Wirtsverbindung, die durch die Chemische Formel I wiedergegeben wird, und eine zweite Wirtsverbindung, die durch die Chemische Formel II wiedergegeben wird: 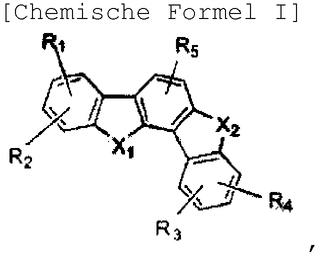
wobei in der Chemischen Formel I
X₁ für O, Se, S, SO, SO₂, PO oder CO steht,
X₂ für CRₐR_{b} oder SiR_{c}R_{d} steht und
R₁ bis R₅ und Rₐ bis R_{d} jeweils unabhängig für Wasserstoff, Deuterium, eine substituierte oder unsubstituierte C1- bis C30-Alkylgruppe, eine substituierte oder unsubstituierte C3- bis C30-Cycloalkylgruppe, eine substituierte oder unsubstituierte C3- bis C30-Heterocycloalkylgruppe, eine substituierte oder unsubstituierte C6- bis C30-Arylgruppe, eine substituierte oder unsubstituierte C3- bis C30-Heteroarylgruppe, eine substituierte oder unsubstituierte Amingruppe, eine substituierte oder unsubstituierte C6- bis C30-Arylamingruppe, eine substituierte oder unsubstituierte C3- bis C30-Heteroarylamingruppe, eine substituierte oder unsubstituierte C1- bis C30-Alkoxygruppe, eine substituierte oder unsubstituierte C2- bis C30-Alkoxycarbonylgruppe, eine substituierte oder unsubstituierte C2- bis C30-Alkoxycarbonylaminogruppe, eine substituierte oder unsubstituierte C7- bis C30-Aryloxycarbonylamino-gruppe, eine substituierte oder unsubstituierte C1- bis C30-Sulfamoylaminogruppe, eine substituierte oder unsubstituierte C2- bis C30-Alkenylgruppe, eine substituierte oder unsubstituierte C2- bis C30-Alkinylgruppe, eine substituierte oder unsubstituierte Silylgruppe, eine substituierte oder unsubstituierte Silyloxygruppe, eine substituierte oder unsubstituierte C1- bis C30-Acylgruppe, eine substituierte oder unsubstituierte C1- bis C20-Acyloxygruppe, eine substituierte oder unsubstituierte C1- bis C20-Acylaminogruppe, eine substituierte oder unsubstituierte C1- bis C30-Sulfonylgruppe, eine substituierte oder unsubstituierte C1- bis C30-Alkylthiolgruppe, eine substituierte oder unsubstituierte C1- bis C30-Heterocyclothiolgruppe, eine substituierte oder unsubstituierte C6- bis C30-Arylthiolgruppe, eine substituierte oder unsubstituierte C1- bis C30-Heteroarylthiolgruppe, eine substituierte oder unsubstituierte C1- bis C30-Ureidgruppe, ein Halogen, eine halogenhaltige Gruppe, eine Cyanogruppe, eine Hydroxylgruppe, eine Aminogruppe, eine Nitrogruppe, eine Carboxylgruppe, eine Ferrocenylgruppe oder eine Kombination davon stehen, 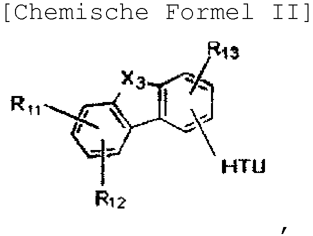
wobei in der Chemischen Formel II
X₃ für O, S, CRᵤRᵥ, SiR_{w}Rₓ oder NR_{y} steht, HTU für eine substituierte oder unsubstituierte Carbazolylgruppe oder eine substituierte oder unsubstituierte Triphenylenylgruppe steht und R₁₁ bis R₁₃ und Rᵤ bis R_{y} jeweils unabhängig für Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C3- bis C30-Heteroarylgruppe oder eine Kombination davon stehen.

2. Zusammensetzung nach Anspruch 1, wobei die erste Wirtsverbindung durch eine der Chemischen Formeln 1a bis 1c wiedergegeben wird: 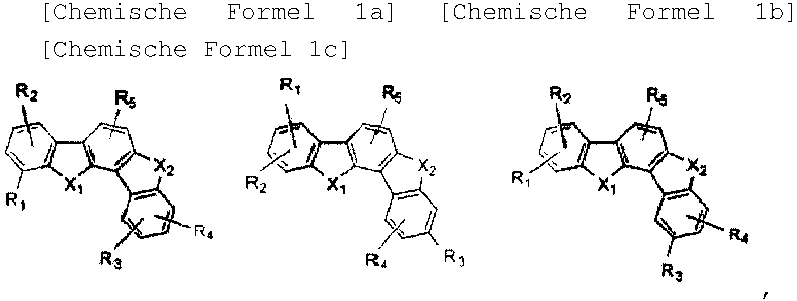
wobei in den Chemischen Formeln 1a bis 1c
X₁ für O, Se, S, SO, SO₂, PO oder CO steht,
X₂ für CRₐR_{b} oder SiR_{c}R_{d} steht und
R₁ bis R₅ und Rₐ bis R_{d} jeweils unabhängig für Wasserstoff, Deuterium, eine substituierte oder unsubstituierte C1- bis C30-Alkylgruppe, eine substituierte oder unsubstituierte C3- bis C30-Cycloalkylgruppe, eine substituierte oder unsubstituierte C3- bis C30-Heterocycloalkylgruppe, eine substituierte oder unsubstituierte C6- bis C30-Arylgruppe, eine substituierte oder unsubstituierte C3- bis C30-Heteroarylgruppe, eine substituierte oder unsubstituierte Amingruppe, eine substituierte oder unsubstituierte C6- bis C30-Arylamingruppe, eine substituierte oder unsubstituierte C3- bis C30-Heteroarylamingruppe, eine substituierte oder unsubstituierte C1- bis C30-Alkoxygruppe, eine substituierte oder unsubstituierte C2- bis C30-Alkoxycarbonylgruppe, eine substituierte oder unsubstituierte C2- bis C30-Alkoxycarbonylaminogruppe, eine substituierte oder unsubstituierte C7- bis C30-Aryloxycarbonylaminogruppe, eine substituierte oder unsubstituierte C1- bis C30-Sulfamoylaminogruppe, eine substituierte oder unsubstituierte C2- bis C30-Alkenylgruppe, eine substituierte oder unsubstituierte C2- bis C30-Alkinylgruppe, eine substituierte oder unsubstituierte Silylgruppe, eine substituierte oder unsubstituierte Silyloxygruppe, eine substituierte oder unsubstituierte C1- bis C30-Acylgruppe, eine substituierte oder unsubstituierte C1- bis C20-Acyloxygruppe, eine substituierte oder unsubstituierte C1- bis C20-Acylaminogruppe, eine substituierte oder unsubstituierte C1- bis C30-Sulfonylgruppe, eine substituierte oder unsubstituierte C1- bis C30-Alkylthiolgruppe, eine substituierte oder unsubstituierte C1- bis C30-Heterocyclothiolgruppe, eine substituierte oder unsubstituierte C6- bis C30-Arylthiolgruppe, eine substituierte oder unsubstituierte C1- bis C30-Heteroarylthiolgruppe, eine substituierte oder unsubstituierte C1- bis C30-Ureidgruppe, ein Halogen, eine halogenhaltige Gruppe, eine Cyanogruppe, eine Hydroxylgruppe, eine Aminogruppe, eine Nitrogruppe, eine Carboxylgruppe, eine Ferrocenylgruppe oder eine Kombination davon stehen.

3. Zusammensetzung nach Anspruch 1, wobei die erste Wirtsverbindung durch eine der Chemischen Formeln 1-1 bis 1-6 wiedergegeben wird: 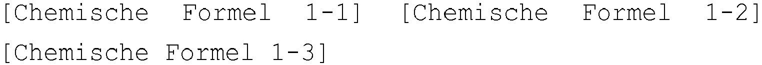 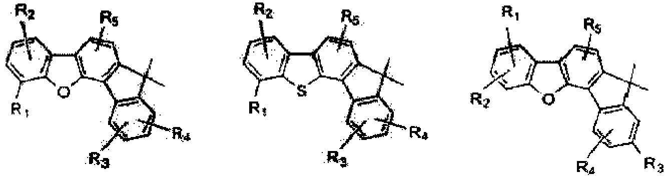 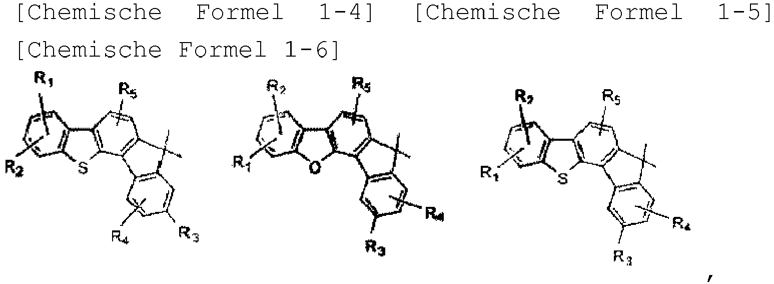 wobei in den Chemischen Formeln 1-1 bis 1-6 R₁ bis R₅ jeweils unabhängig für Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C3- bis C30-Heteroarylgruppe oder eine Kombination davon stehen.

4. Zusammensetzung nach Anspruch 1, wobei mindestens eine der Variablen R₁ bis R₅ und Rₐ bis R_{d} aus substituierten oder unsubstituierten funktionellen Gruppen der Gruppe 1 ausgewählt ist: 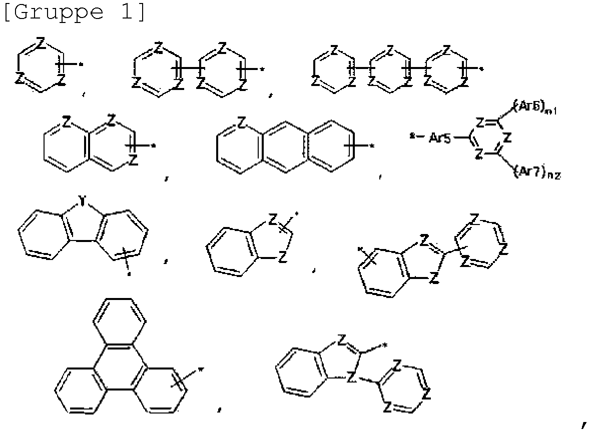 wobei in der Gruppe 1
Y für N, O, S, SO₂, NRⱼ, CRₖ, SiRₗ, CRₘRₙ oder SiRₒRₚ steht,
Z für N, CR_{q}, CRᵣRₛ oder NRₜ steht,
wobei Rⱼ bis Rₜ jeweils unabhängig für Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C3- bis C30-Heteroarylgruppe oder eine Kombination davon stehen,
Ar5 für eine Einfachbindung, eine substituierte oder unsubstituierte C1- bis C20-Alkylengruppe, eine substituierte oder unsubstituierte C2- bis C20-Alkenylengruppe, eine substituierte oder unsubstituierte C6- bis C30-Arylengruppe, eine substituierte oder unsubstituierte C2- bis C30-Heteroarylengruppe, eine substituierte oder unsubstituierte C6- bis C30-Arylamingruppe, eine substituierte oder unsubstituierte C2- bis C30-Heteroarylamingruppe oder eine Kombination davon steht,
Ar6 und Ar7 unabhängig für eine substituierte oder unsubstituierte C6- bis C30-Arylgruppe, eine substituierte oder unsubstituierte C3- bis C30-Heteroarylgruppe oder eine Kombination davon stehen,
n1 und n2 jeweils unabhängig für 0 oder 1 stehen und
* ein Verknüpfungspunkt zur Chemischen Formel 1 ist und sich an einem der Elemente der funktionellen Gruppe befinden kann.

5. Zusammensetzung nach Anspruch 4, wobei die substituierte oder unsubstituierte funktionelle Gruppe von Gruppe 1 aus den substituierten oder unsubstituierten Chemischen Formeln B-1 bis B-35 ausgewählt ist:

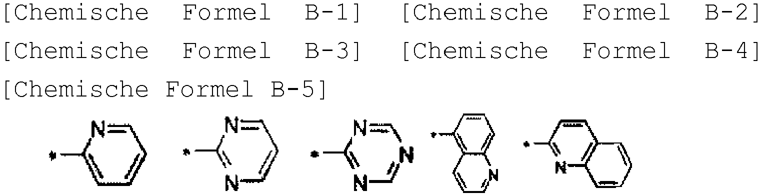

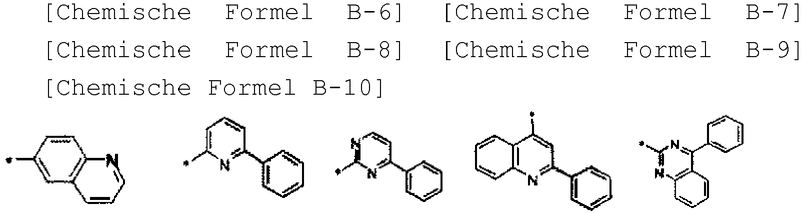

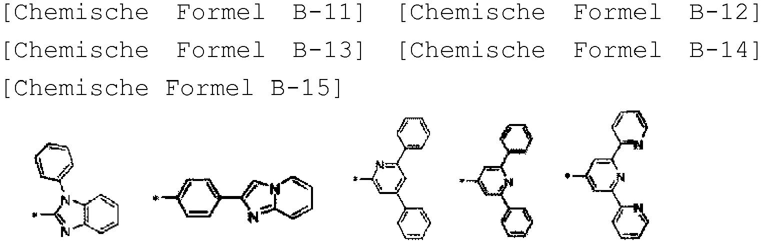

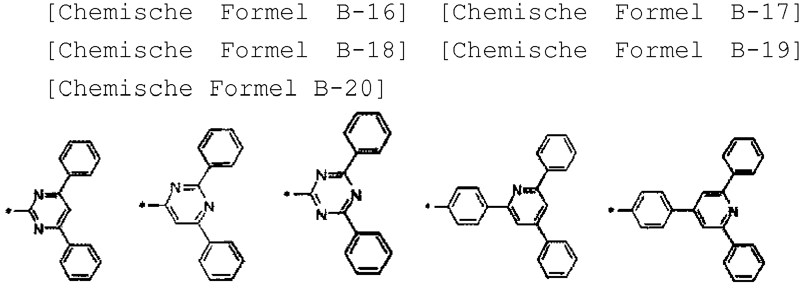

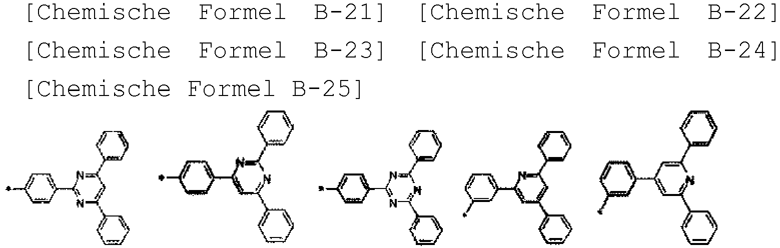

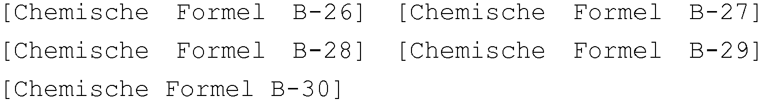

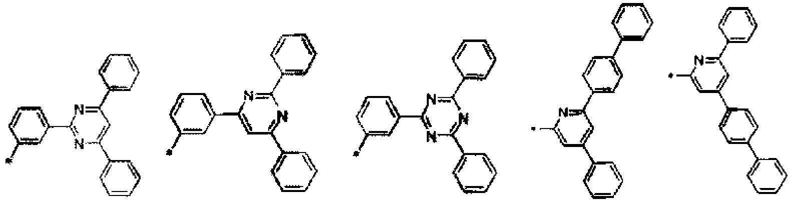

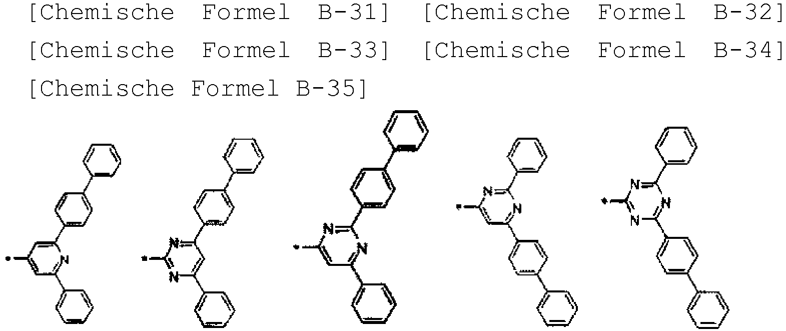

6. Zusammensetzung nach Anspruch 1, wobei die erste Wirtsverbindung aus den Chemischen Formeln 1-A bis 1-P ausgewählt ist:

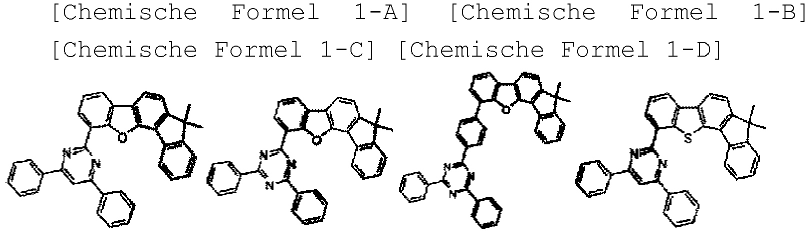

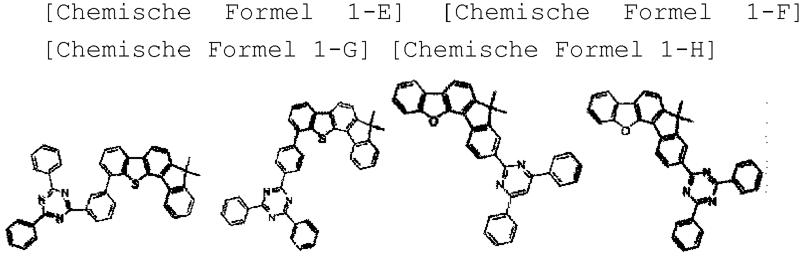

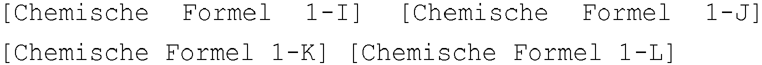

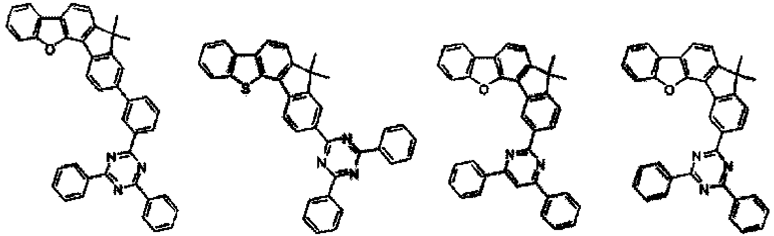

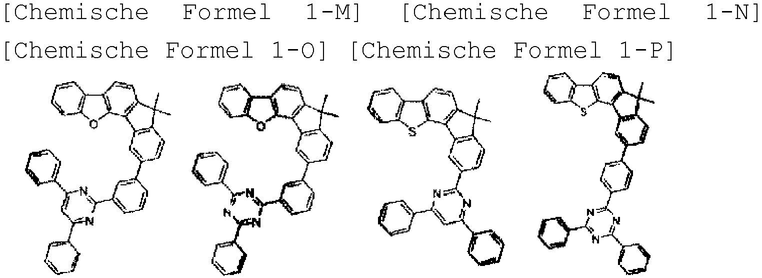

7. Zusammensetzung nach Anspruch 1, wobei die zweite Wirtsverbindung durch die Chemische Formel 2 wiedergegeben wird: 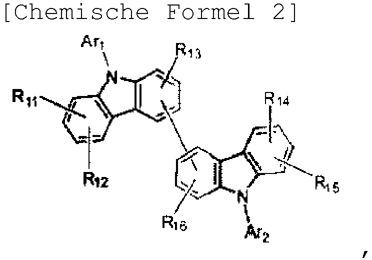
wobei in der Chemischen Formel 2
Ar₁, Ar₂ und R₁₁ bis R₁₆ jeweils unabhängig für Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C3- bis C30-Heteroarylgruppe oder eine Kombination davon stehen.

8. Zusammensetzung nach Anspruch 7, wobei die zweite Wirtsverbindung durch eine der Chemischen Formeln 2-1 bis 2-3 wiedergegeben wird: 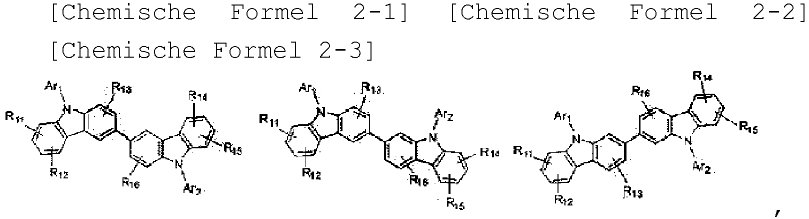 wobei in den Chemischen Formeln 2-1 bis 2-3 Ar₁, Ar₂ und R₁₁ bis R₁₆ jeweils unabhängig für Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C3- bis C30-Heteroarylgruppe oder eine Kombination davon stehen.

9. Zusammensetzung nach Anspruch 7, wobei Ar₁ und Ar₂ der Chemischen Formel 2 jeweils unabhängig aus den substituierten oder unsubstituierten Chemischen Formeln A-1 bis A-15 ausgewählt sind:

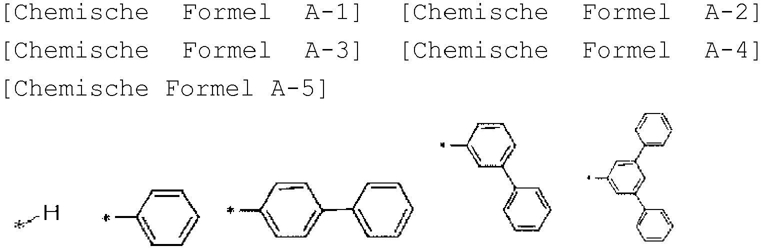

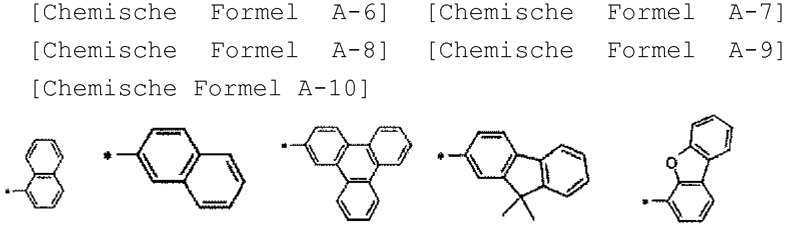

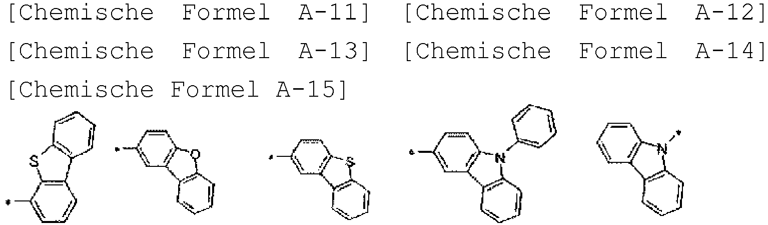

10. Zusammensetzung nach Anspruch 1, wobei die zweite Wirtsverbindung durch die Chemische Formel 3 wiedergegeben wird: 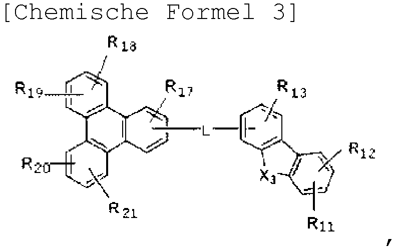
wobei in der Chemischen Formel 3
X₃ für O, S, CRₑR_{f}, SiR_{g}Rₕ oder NRᵢ steht,
L für eine Einfachbindung, eine substituierte oder unsubstituierte C1- bis C20-Alkylengruppe, eine substituierte oder unsubstituierte C2- bis C20-Alkenylengruppe, eine substituierte oder unsubstituierte C6- bis C30-Arylengruppe, eine substituierte oder unsubstituierte C2- bis C30-Heteroarylengruppe oder eine Kombination davon steht und
R₁₁ bis R₁₃, R₁₇ bis R₂₁ und Rₑ bis Rᵢ jeweils unabhängig für Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C3- bis C30-Heteroarylgruppe oder eine Kombination davon stehen.

11. Zusammensetzung nach Anspruch 10, wobei die zweite Wirtsverbindung durch eine der Chemischen Formeln 3-1 bis 3-10 wiedergegeben wird: 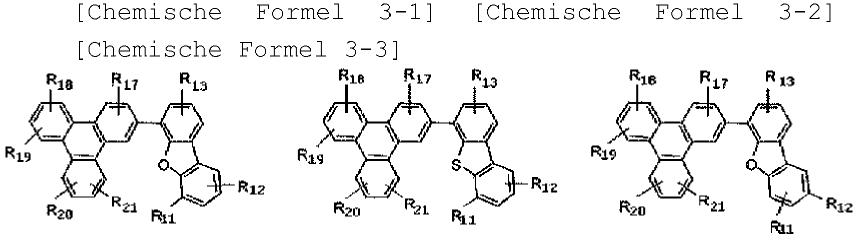 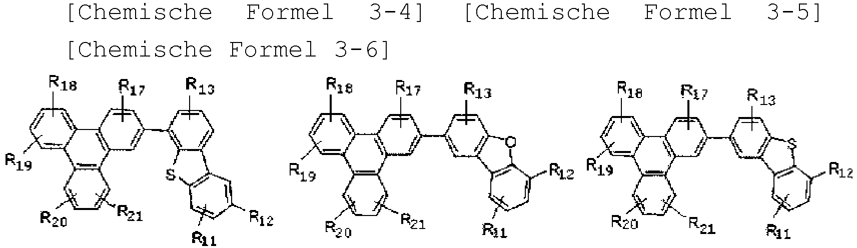 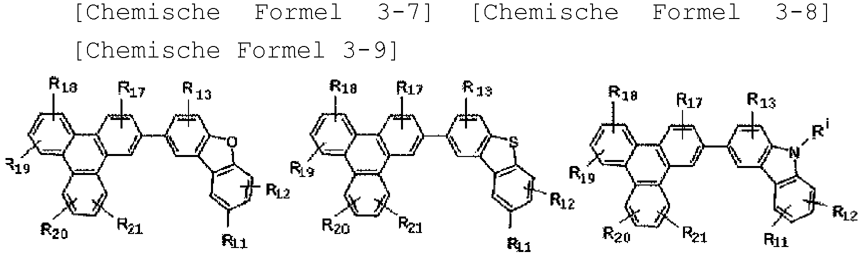 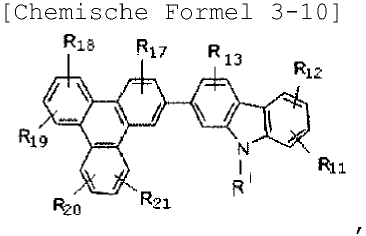
wobei in den Chemischen Formeln 3-1 bis 3-10
R₁₁ bis R₁₃, R₁₇ bis R₂₁ und Rᵢ jeweils unabhängig für Wasserstoff, Deuterium, eine substituierte oder unsubstituierte C1- bis C20-Alkylgruppe, eine substituierte oder unsubstituierte C6- bis C30-Arylgruppe, eine substituierte oder unsubstituierte C3- bis C30-Heteroarylgruppe oder eine Kombination davon stehen.

12. Zusammensetzung nach Anspruch 10, wobei mindestens eine der Variablen R₁₁ bis R₁₃, R₁₇ bis R₂₁ und Rₑ bis Rᵢ aus den substituierten oder unsubstituierten Chemischen Formeln A-1 bis A-15 ausgewählt ist:

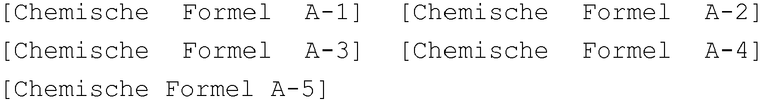

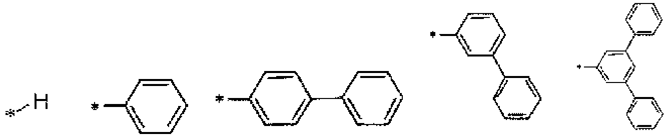

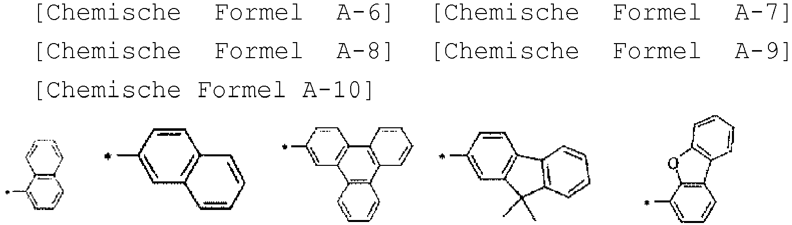

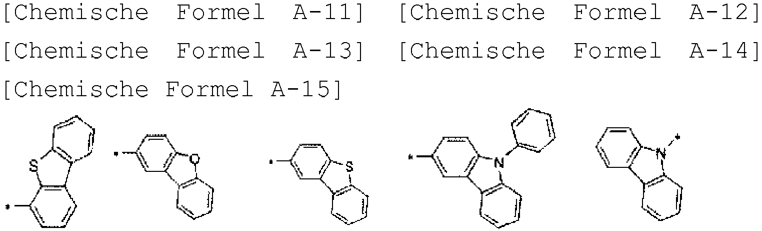

13. Zusammensetzung nach Anspruch 1, wobei die zweite Wirtsverbindung durch die Chemischen Formeln 2-A bis 2-C oder die Chemischen Formeln 3-A bis 3-D oder 3-E wiedergegeben wird:

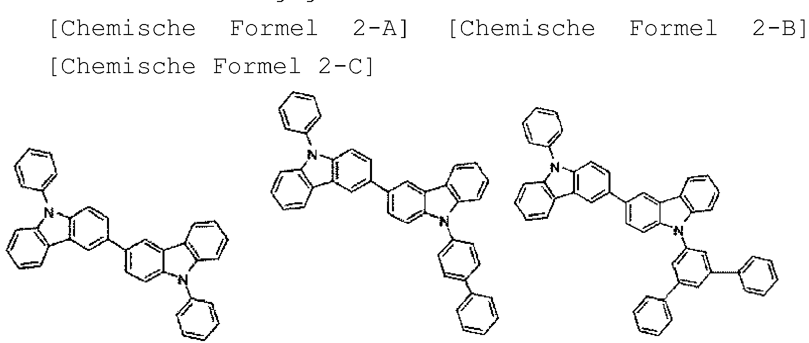

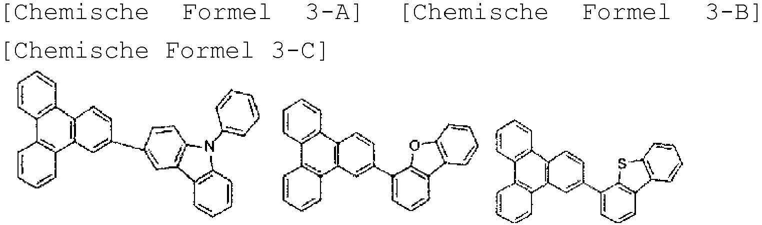

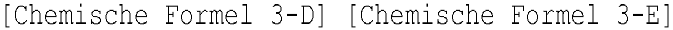

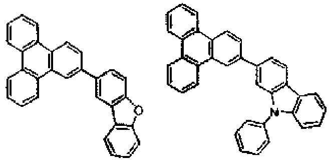

14. Zusammensetzung nach Anspruch 1, wobei die erste Wirtsverbindung und die zweite Wirtsverbindung in einem Gewichtsverhältnis von 1:10 bis 10:1 enthalten sind.

15. Organische optoelektronische Vorrichtung, umfassend
eine Anode und eine Kathode, die einander zugewandt sind, und
mindestens eine organische Schicht, die zwischen der Anode und der Kathode angeordnet ist,
wobei die organische Schicht die Zusammensetzung nach einem der Ansprüche 1 bis 14 umfasst.

## Revendications

1. Composition comprenant un premier composé hôte représenté par la formule chimique I et un deuxième composé hôte représenté par la formule chimique II : 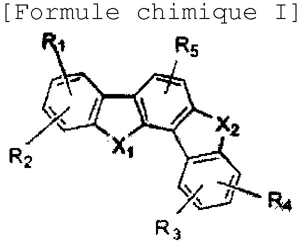
dans laquelle, dans la formule chimique I,
X₁ est O, Se, S, SO, SO₂, PO ou CO,
X₂ est CRₐR_{b} ou SiR_{c}R_{d}, et
R₁ à R₅ et Rₐ à R_{d} sont chacun indépendamment hydrogène, deutérium, un groupe alkyle en C1 à C30 substitué ou non substitué, un groupe cycloalkyle en C3 à C30 substitué ou non substitué, un groupe hétérocycloalkyle en C3 à C30 substitué ou non substitué, un groupe aryle en C6 à C30 substitué ou non substitué, un groupe hétéroaryle en C3 à C30 substitué ou non substitué, un groupe amine substitué ou non substitué, un groupe arylamine en C6 à C30 substitué ou non substitué, un groupe hétéroarylamine en C3 à C30 substitué ou non substitué, un groupe alcoxy en C1 à C30 substitué ou non substitué, un groupe alcoxycarbonyle en C2 à C30 substitué ou non substitué, un groupe alcoxycarbonylamino en C2 à C30 substitué ou non substitué, un groupe aryloxycarbonylamino en C7 à C30 substitué ou non substitué, un groupe sulfamoylamino en C1 à C30 substitué ou non substitué, un groupe alcényle en C2 à C30 substitué ou non substitué, un groupe alcynyle en C2 à C30 substitué ou non substitué, un groupe silyle substitué ou non substitué, un groupe silyloxy substitué ou non substitué, un groupe acyle en C1 à C30 substitué ou non substitué, un groupe acyloxy en C1 à C20 substitué ou non substitué, un groupe acylamino en C1 à C20 substitué ou non substitué, un groupe sulfonyle en C1 à C30 substitué ou non substitué, un groupe alkylthiol en C1 à C30 substitué ou non substitué, un groupe hétérocyclothiol en C1 à C30 substitué ou non substitué, un groupe arylthiol en C6 à C30 substitué ou non substitué, un groupe hétéroarylthiol en C1 à C30 substitué ou non substitué, un groupe uréide en C1 à C30 substitué ou non substitué, un halogène, un groupe contenant un halogène, un groupe cyano, un groupe hydroxyle, un groupe amino, un groupe nitro, un groupe carboxyle, un groupe ferrocényle, ou une combinaison de ceux-ci, 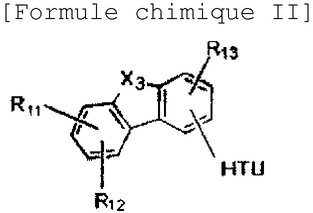
dans laquelle, dans la formule chimique II,
X₃ est O, S, CRᵤRᵥ, SiR_{w}Rₓ ou NR_{y},
HTU est un groupe carbazolyle substitué ou non substitué ou un groupe triphénylényle substitué ou non substitué, et
R₁₁ à R₁₃ et Rᵤ à R_{y} sont chacun indépendamment hydrogène, deutérium, un groupe alkyle en C1 à C20 substitué ou non substitué, un groupe aryle en C6 à C30 substitué ou non substitué, un groupe hétéroaryle en C3 à C30 substitué ou non substitué, ou une combinaison de ceux-ci.

2. Composition de la revendication 1, dans laquelle le premier composé hôte est représenté par une des formules chimiques 1a à 1c : 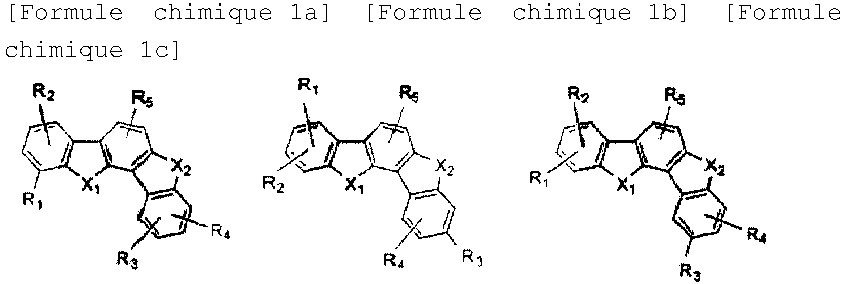
dans laquelle, dans les formules chimiques 1a à 1c,
X₁ est O, Se, S, SO, SO₂, PO ou CO,
X₂ est CRₐR_{b} ou SiR_{c}R_{d}, et
R₁ à R₅ et Rₐ à R_{d} sont chacun indépendamment hydrogène, deutérium, un groupe alkyle en C1 à C30 substitué ou non substitué, un groupe cycloalkyle en C3 à C30 substitué ou non substitué, un groupe hétérocycloalkyle en C3 à C30 substitué ou non substitué, un groupe aryle en C6 à C30 substitué ou non substitué, un groupe hétéroaryle en C3 à C30 substitué ou non substitué, un groupe amine substitué ou non substitué, un groupe arylamine en C6 à C30 substitué ou non substitué, un groupe hétéroarylamine en C3 à C30 substitué ou non substitué, un groupe alcoxy en C1 à C30 substitué ou non substitué, un groupe alcoxycarbonyle en C2 à C30 substitué ou non substitué, un groupe alcoxycarbonylamino en C2 à C30 substitué ou non substitué, un groupe aryloxycarbonylamino en C7 à C30 substitué ou non substitué, un groupe sulfamoylamino en C1 à C30 substitué ou non substitué, un groupe alcényle en C2 à C30 substitué ou non substitué, un groupe alcynyle en C2 à C30 substitué ou non substitué, un groupe silyle substitué ou non substitué, un groupe silyloxy substitué ou non substitué, un groupe acyle en C1 à C30 substitué ou non substitué, un groupe acyloxy en C1 à C20 substitué ou non substitué, un groupe acylamino en C1 à C20 substitué ou non substitué, un groupe sulfonyle en C1 à C30 substitué ou non substitué, un groupe alkylthiol en C1 à C30 substitué ou non substitué, un groupe hétérocyclothiol en C1 à C30 substitué ou non substitué, un groupe arylthiol en C6 à C30 substitué ou non substitué, un groupe hétéroarylthiol en C1 à C30 substitué ou non substitué, un groupe uréide en C1 à C30 substitué ou non substitué, un halogène, un groupe contenant un halogène, un groupe cyano, un groupe hydroxyle, un groupe amino, un groupe nitro, un groupe carboxyle, un groupe ferrocényle, ou une combinaison de ceux-ci.

3. Composition de la revendication 1, dans laquelle le premier composé hôte est représenté par une des formules chimiques 1-1 à 1-6 : 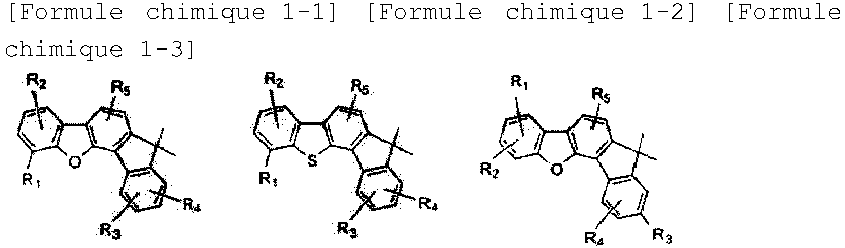 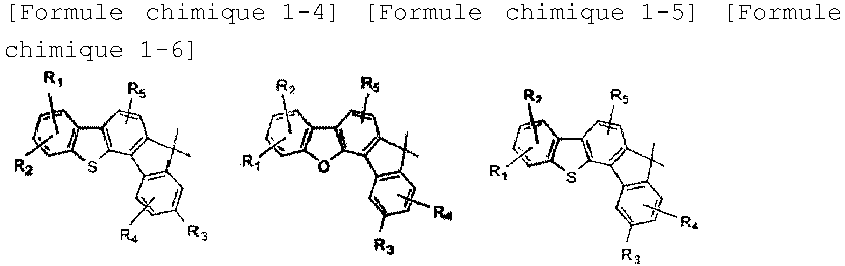 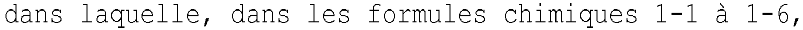 R₁ à R₅ sont chacun indépendamment hydrogène, deutérium, un groupe alkyle en C1 à C20 substitué ou non substitué, un groupe aryle en C6 à C30 substitué ou non substitué, un groupe hétéroaryle en C3 à C30 substitué ou non substitué, ou une combinaison de ceux-ci.

4. Composition de la revendication 1, dans laquelle au moins l'un de R₁ à R₅ et Rₐ à R_{d} est choisi parmi des groupes fonctionnels substitués ou non substitués du groupe 1 : 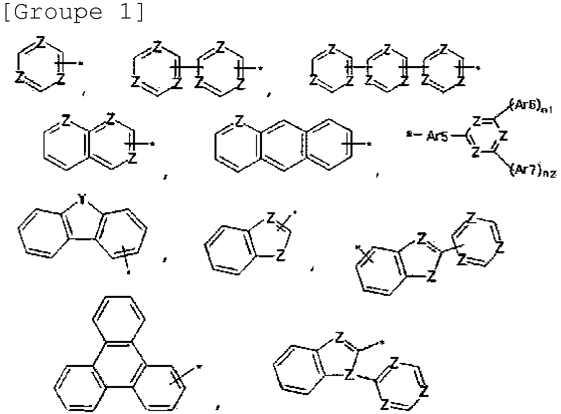
dans laquelle, dans le groupe 1,
Y est N, O, S, SO₂, NRⱼ, CRₖ, SiRₗ, CRₘRₙ ou SiRₒRₚ,
Z est N, CR_{q}, CRᵣRₛ ou NRₜ,
dans laquelle Rⱼ à Rₜ sont chacun indépendamment hydrogène, deutérium, un groupe alkyle en C1 à C20 substitué ou non substitué, un groupe aryle en C6 à C30 substitué ou non substitué, un groupe hétéroaryle en C3 à C30 substitué ou non substitué, ou une combinaison de ceux-ci,
Ar5 est une simple liaison, un groupe alkylène en C1 à C20 substitué ou non substitué, un groupe alcénylène en C2 à C20 substitué ou non substitué, un groupe arylène en C6 à C30 substitué ou non substitué, un groupe hétéroarylène en C2 à C30 substitué ou non substitué, un groupe arylamine en C6 à C30 substitué ou non substitué, un groupe hétéroarylamine en C2 à C30 substitué ou non substitué, ou une combinaison de ceux-ci,
Ar₆ et Ar₇ sont indépendamment un groupe aryle en C6 à C30 substitué ou non substitué, un groupe hétéroaryle en C3 à C30 substitué ou non substitué, ou une combinaison de ceux-ci,
n1 et n2 sont chacun indépendamment 0 ou 1, et
* est un point de liaison à la formule chimique 1 et peut être positionné à un des éléments du groupe fonctionnel.

5. Composition de la revendication 4, dans laquelle le groupe fonctionnel substitué ou non substitué du groupe 1 est choisi parmi les formules chimiques B-1 à B-35 substituées ou non substituées :

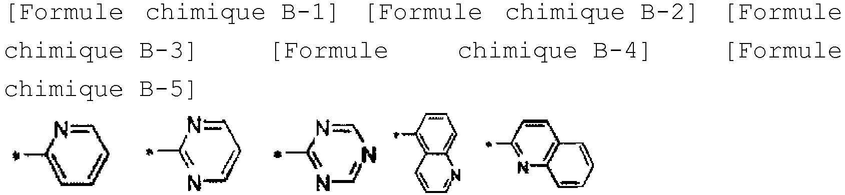

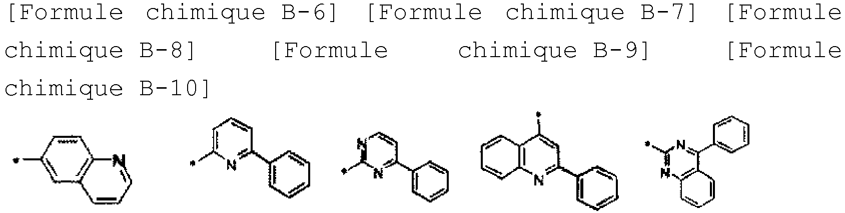

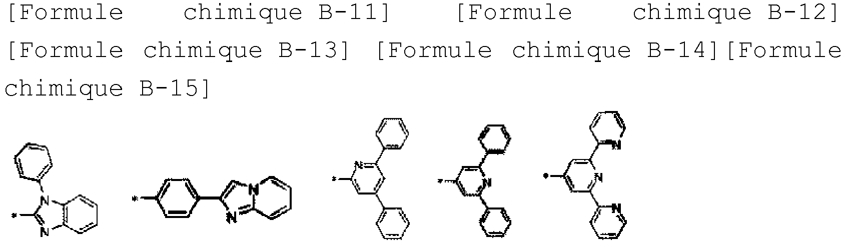

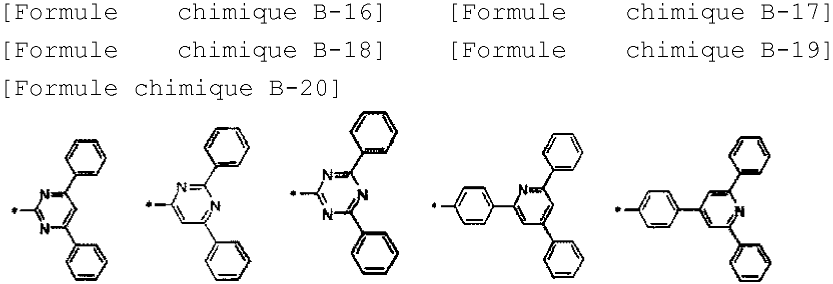

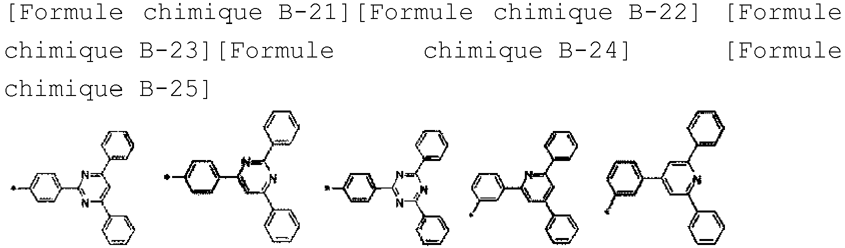

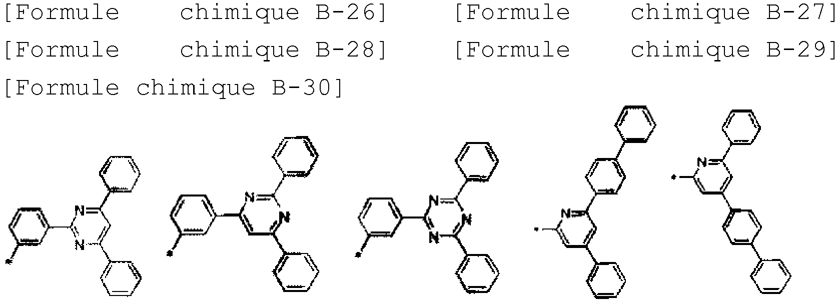

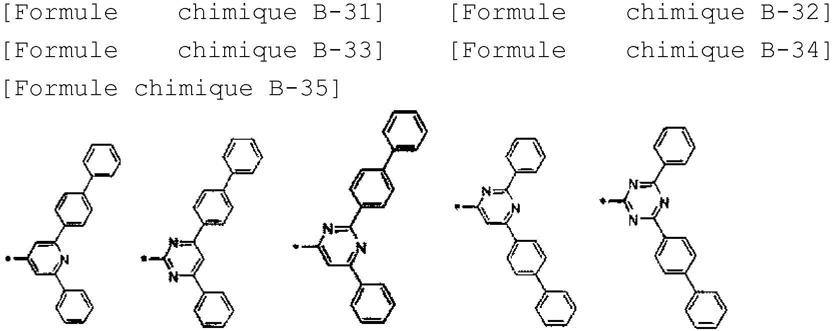

6. Composition de la revendication 1, dans laquelle le premier composé hôte est choisi parmi les formules chimiques 1-A à 1-P :

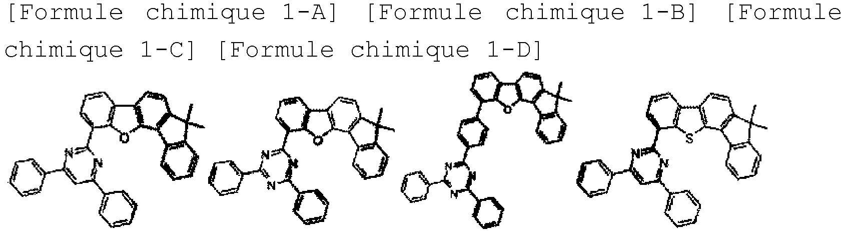

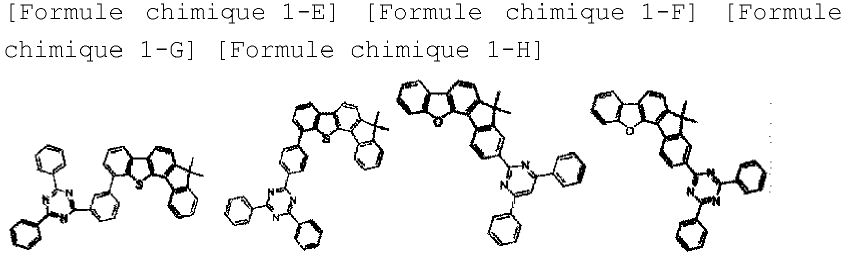

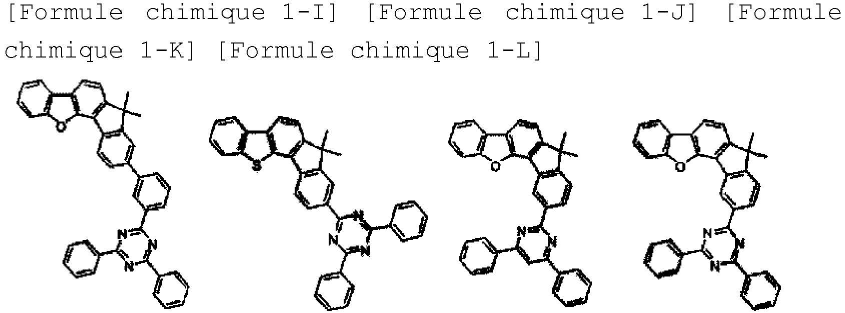

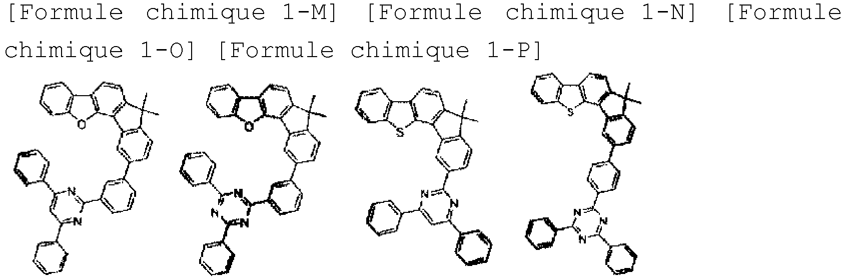

7. Composition de la revendication 1, dans laquelle le deuxième composé hôte est représenté par la formule chimique 2 : 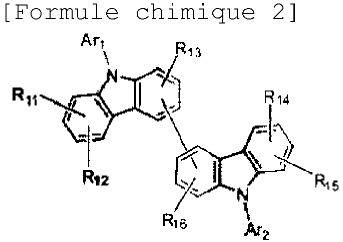
dans laquelle, dans la formule chimique 2,
Ar₁, Ar₂ et R₁₁ à R₁₆ sont chacun indépendamment hydrogène, deutérium, un groupe alkyle en C1 à C20 substitué ou non substitué, un groupe aryle en C6 à C30 substitué ou non substitué, un groupe hétéroaryle en C3 à C30 substitué ou non substitué, ou une combinaison de ceux-ci.

8. Composition de la revendication 7, dans laquelle le deuxième composé hôte est représenté par une des formules chimiques 2-1 à 2-3 : 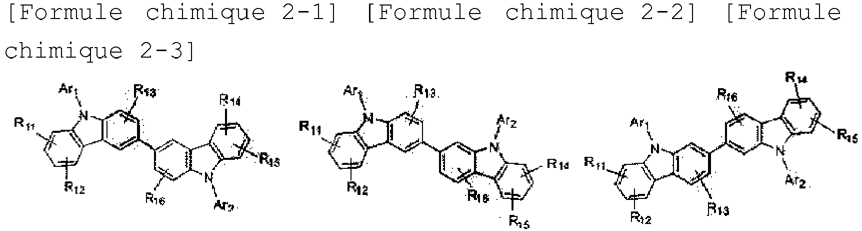
dans laquelle, dans les formules chimiques 2-1 à 2-3,
Ar₁, Ar₂ et R₁₁ à R₁₆ sont chacun indépendamment hydrogène, deutérium, un groupe alkyle en C1 à C20 substitué ou non substitué, un groupe aryle en C6 à C30 substitué ou non substitué, un groupe hétéroaryle en C3 à C30 substitué ou non substitué, ou une combinaison de ceux-ci.

9. Composition de la revendication 7, dans laquelle Ar₁ et Ar₂ de formule chimique 2 sont chacun indépendamment choisi parmi des formules chimiques A-1 à A-15 substituées ou non substituées :

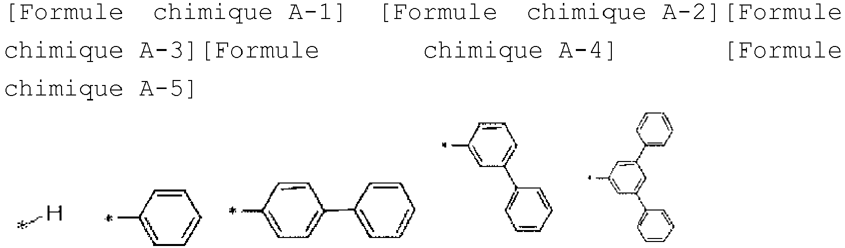

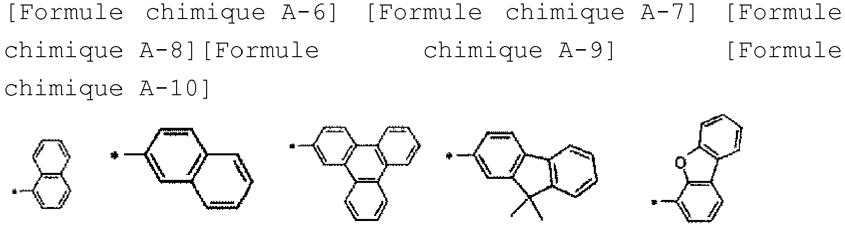

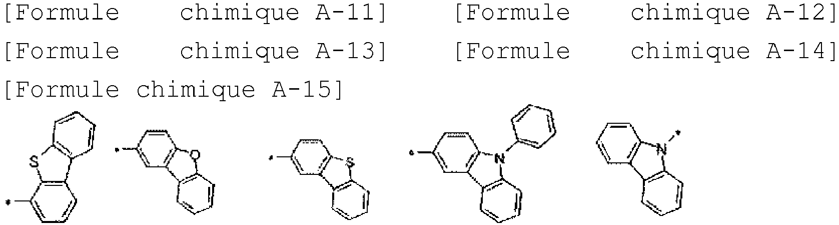

10. Composition de la revendication 1, dans laquelle le deuxième composé hôte est représenté par la formule chimique 3 : 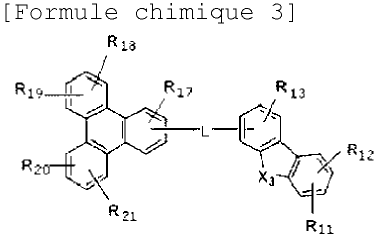 dans laquelle, dans la formule chimique 3,
X₃ est O, S, CRₑR_{f}, SiR_{g}Rₕ ou NRᵢ,
L est une simple liaison, un groupe alkylène en C1 à C20 substitué ou non substitué, un groupe alcénylène en C2 à C20 substitué ou non substitué, un groupe arylène en C6 à C30 substitué ou non substitué, un groupe hétéroarylène en C2 à C30 substitué ou non substitué, ou une combinaison de ceux-ci, et
R₁₁ à R₁₃, R₁₇ à R₂₁, et Rₑ à Rᵢ sont chacun indépendamment hydrogène, deutérium, un groupe alkyle en C1 à C20 substitué ou non substitué, un groupe aryle en C6 à C30 substitué ou non substitué, un groupe hétéroaryle en C3 à C30 substitué ou non substitué, ou une combinaison de ceux-ci.

11. Composition de la revendication 10, dans laquelle le deuxième composé hôte est représenté par une des formules chimiques 3-1 à 3-10 : 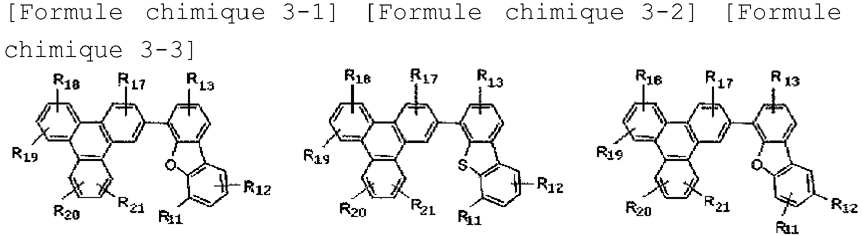 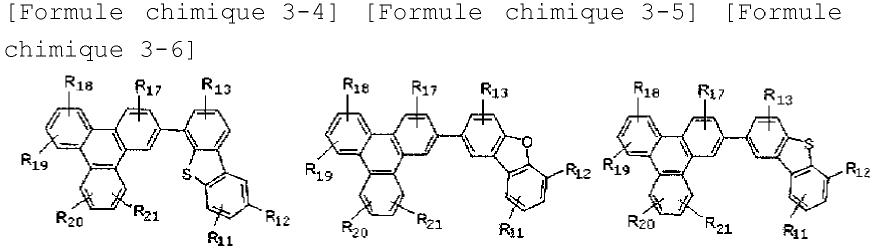 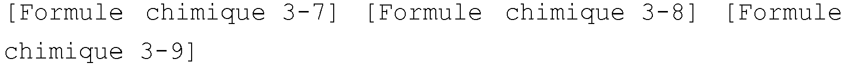 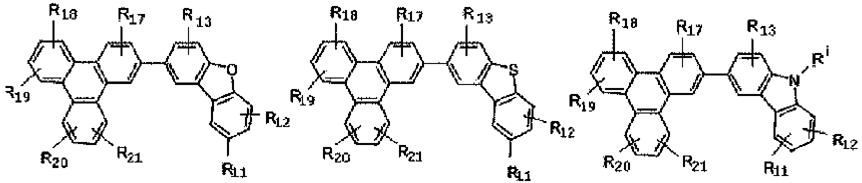 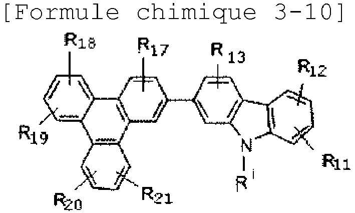
dans laquelle, dans les formules chimiques 3-1 à 3-10,
R₁₁ à R₁₃, R₁₇ à R₂₁, et Rᵢ sont chacun indépendamment hydrogène, deutérium, un groupe alkyle en C1 à C20 substitué ou non substitué, un groupe aryle en C6 à C30 substitué ou non substitué, un groupe hétéroaryle en C3 à C30 substitué ou non substitué, ou une combinaison de ceux-ci.

12. Composition de la revendication 10, dans laquelle au moins l'un de R₁₁ à R₁₃, R₁₇ à R₂₁, et Rₑ à Rᵢ est choisi parmi les formules chimiques A-1 à A-15 substituées ou non substituées,

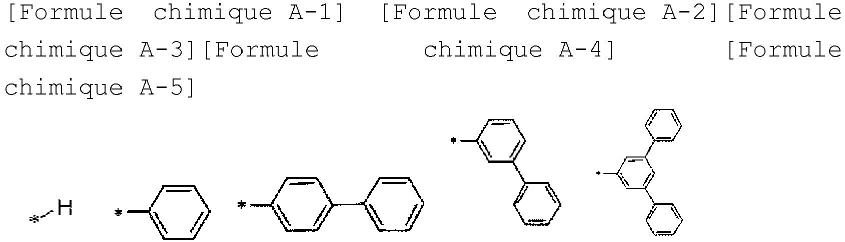

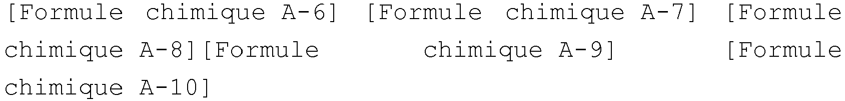

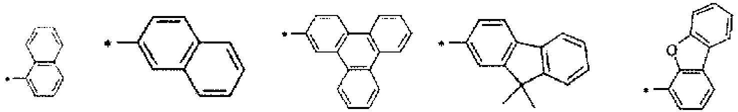

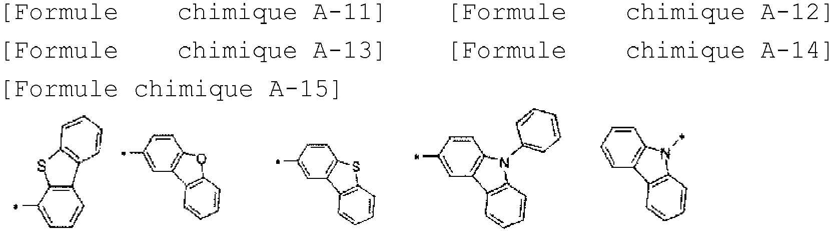

13. Composition de la revendication 1, dans laquelle le deuxième composé hôte est représenté par les formules chimiques 2-A à 2-C, formules chimiques 3-A à 3-D ou 3-E :

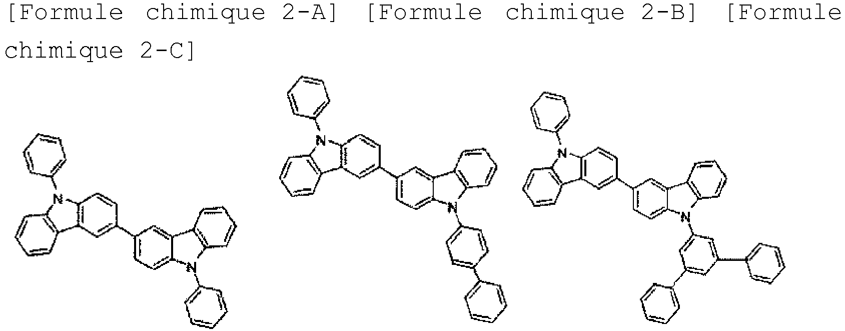

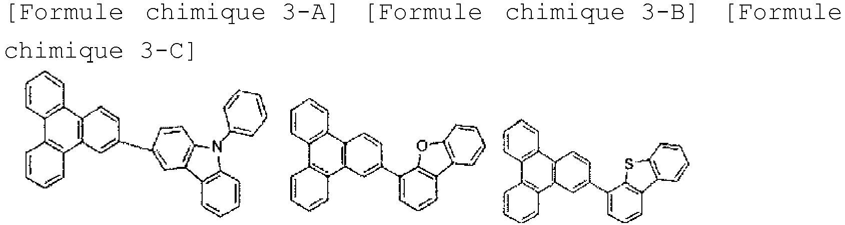

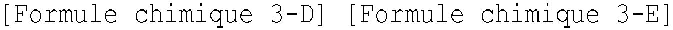

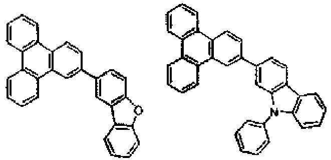

14. Composition de la revendication 1, dans laquelle le premier composé hôte et le deuxième composé hôte sont inclus dans un rapport en poids de 1:10 à 10:1.

15. Dispositif opto-électrique organique comprenant une anode et une cathode se faisant mutuellement face, et
au moins une couche organique intercalée entre l'anode et la cathode, dans lequel la couche organique comprend la composition de l'une quelconque de la revendication 1 à la revendication 14.
